(19) Europäisches Patentamt
European Patent Office
Office européen des brevets

(11) **EP 0 655 462 B1**

(12) **EUROPÄISCHE PATENTSCHRIFT**

(45) Veröffentlichungstag und Bekanntmachung des Hinweises auf die Patenterteilung:
06.05.1998 Patentblatt 1998/19

(51) Int Cl.⁶: **C07K 14/46**, C07K 14/75, A61K 38/04

(21) Anmeldenummer: 94116556.5

(22) Anmeldetag: 20.10.1994

(54) **Lineare Adhäsionsinhibitoren**

Linear adhesion inhibitors

Inhibiteur d'adhésion linéares

(84) Benannte Vertragsstaaten:
AT BE CH DE DK ES FR GB GR IE IT LI LU NL PT SE

(30) Priorität: 28.10.1993 DE 4336758

(43) Veröffentlichungstag der Anmeldung:
31.05.1995 Patentblatt 1995/22

(73) Patentinhaber: MERCK PATENT GmbH
64293 Darmstadt (DE)

(72) Erfinder:
• Jonczyk, Alfred, Dr.
D-64295 Darmstadt (DE)
• Felding-Habermann, Brunhilde, Dr., c/o BFH, SBR 8
La Jolla, CA 92037 (US)
• Diefenbach, Beate
D-64289 Darmstadt (DE)
• Rippmann, Friedrich, Dr.
D-69120 Heidelberg (DE)

(56) Entgegenhaltungen:
EP-A- 0 382 538          WO-A- /15072
US-A- 2 799 670          US-A- 4 177 277

• JOURNAL OF BIOLOGICAL CHEMISTRY., Bd.263, Nr.36, 25. Dezember 1988, BALTIMORE US Seiten 19827 - 19832 ZHONG-RU GHAN ET AL. 'Echistatin. A potent platelet aggregation inhibitor from the venom of the viper, Echis carenatus'

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft neue lineare Peptide der Formel I mit einer Mindestlänge von 4 Aminosäureresten

$$X\text{-}A\text{-}Cys(R^1)\text{-}B\text{-}Z \qquad\qquad I,$$

die aus der C-terminalen Sequenz des Echistatins abgeleitet wurden und
worin

X   H oder Ac,

A   fehlt, Asp oder ein Peptidfragment, ausgewählt aus einer Gruppe bestehend aus Ala-Asp, Thr-Ala-Asp, Lys-Thr-Ala-Asp, Lys-Thr-Ala-Asn, Lys-Thr-Gly-Asp, Lys-Ala-Ala-Asp, Arg-Thr-Ala-Asp, Ser-Ala-Asp, Gln-Ser-Ala-Asp, Gly-Lys-Thr-Ala-Asp, Asn-Gly-Lys-Thr-Ala-Asp, Ile-Ser-Ala-Gly, Arg-Ser-Ala-Gly, Cys-Asn-Gly-Lys-Thr-Ala-Asp; Tyr-Cys-Asn-Gly-Lys-Thr-Ala-Asp, Asp-Tyr-Cys-Asn-Gly-Lys-Thr-Ala-Asp, Gly-Lys-Thr-Cys-Asp, Asp-Asp-Tyr-Cys-Asn-Gly-Lys-Thr-Ala-Asp, Gly-Lys-Thr-Cys(Trt)-Asp, Met-Asp-Asp-Tyr-Cys-Asn-Gly-Lys-Thr-Ala-Asp, Asp-Met-Asp-Asp-Tyr-Cys-Asn-Gly-Lys-Thr-Ala-Asp,

B   fehlt, Ala, Arg, Asn, Asp, Cys, Gln, Glu, Gly, His, Ile, Leu, Lys, Met, Orn, Phe, 4-Hal-Phe, Pro, Ser, Thr, Trp, Tyr, Val oder ein N-methyliertes Derivat der genannten Aminosäurereste, oder ein Peptidfragment, ausgewählt aus einer Gruppe bestehend aus Pro-Arg, Pro-Arg-Asn, Pro-Arg-Asn-Pro, Pro-Arg-Asn-Pro-His, Pro-Arg-Asn-Pro-His-Lys, Pro-Arg-Asn-Pro-His-Lys-Gly, Pro-Arg-Asn-Pro-His-Lys-Gly-Pro, Pro-Arg-Asn-Pro-His-Lys-Gly-Pro-Ala, Pro-Arg-Asn-Pro-His-Lys-Gly-Pro-Ala-Thr,

wobei lediglich einer der Reste A oder B fehlen kann,

Z     OH, OR$^2$, NH$_2$, NHR$^2$ oder N(R$^2$)$_2$,
R$^1$     H, R$^2$, Trt, Dpm oder Bzl,
R$^2$     Alkyl mit 1-6 C-Atomen
Hal     F, Cl, Br oder I

und

Ac    Alkanoyl mit 1-10 C-Atomen, Aralkanoyl mit 8-10 C-Atomen oder Aroyl mit 7-11 C-Atomen

bedeuten,
sowie deren physiologisch unbedenkliche Salze.

Ähnliche Verbindungen sind beispielsweise aus der europäischen Patentanmeldung EP 0 406 428 bekannt. Die vorliegenden Verbindungen leiten sich vom Echistatin, einem Schlangengift, aus dessen C-terminalen Sequenz ab. Echistatin wird in J. Biol. Chem. 2636 19827-19832 (1988) beschrieben. Echistatin ist ein nicht-selektiv wirkender Adhäsionsinhibitor.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem wirken sie als Integrin-Inhibitoren, wobei sie insbesondere die Wechselwirkungen der β$_3$-Integrin-Rezeptoren mit Liganden hemmen. Diese Wirkung kann z.B. nach der Methode nachgewiesen werden, die von J.W. Smith et al. in J. Biol. Chem. 265, 12267-12271 (1990) beschrieben wird. Zusätzlich treten antiinflammatorische Effekte auf. Auch diese Wirkung kann mit Hilfe von literaturbekannten Methoden nachgewiesen werden.

Die Verbindungen können als Arzneimittelwirkstoffe in der Human- und Veterinärmedizin eingesetzt werden, insbesondere zur Prophylaxe und zur Behandlung von Erkrankungen des Kreislaufs, bei Thrombose, Herzinfarkt, coronaren Herzerkrankungen, Arterio- und Atherosklerose, Entzündungen, Apoplexie, Angina pectoris, Tumoren, osteolytischen Erkrankungen, insbesondere Osteoporose, Angiogenese und Restenose nach Angioplastie. Ferner können sie unterstützend bei Wundheilungsprozessen wirken.

Die vor- und nachstehend aufgeführten Abkürzungen von Aminosäureresten stehen für die Reste folgender Aminosäuren:

| Ala | Alanin |
|-----|--------|
| Arg | Arginin |
| Asn | Asparagin |
| Asp | Asparaginsäure |
| Arg | Arginin |
| Cys | Cystein |
| Gln | Glutamin |
| Glp | Pyroglutamin |
| Glu | Glutaminsäure |
| Gly | Glycin |
| His | Histidin |
| Ile | Isoleucin |
| Leu | Leucin |
| Lys | Lysin |
| Met | Methionin |
| Orn | Ornithin |
| Phe | Phenylalanin |
| Pro | Prolin |
| Ser | Serin |
| Thr | Threonin |
| Trp | Tryptophan |
| Tyr | Tyrosin |
| Val | Valin. |

Ferner bedeuten nachstehend:

| BOC | tert-Butoxycarbonyl |
|-----|---------------------|
| Bzl | Benzyl |
| CBZ | Benzyloxycarbonyl |
| DCCI | Dicyclohexylcarbodiimid |
| Dpm | Diphenylmethyl |
| DMF | Dimethylformamid |
| EDCI | N-Ethyl-N'-(3-dimethylaminopropyl)-carbodiimidhydrochlorid |
| Et | Ethyl |
| $Et_2O$ | Diethylether |
| Fmoc | 9-Fluorenylmethoxycarbonyl |
| HOBt | 1-Hydroxybenzotriazol |
| Me | Methyl |
| MBHA | 4-Methyl-benzhydrylamin |
| Mtr | 4-Methoxy-2,3,6-trimethylphenyl-sulfonyl |
| OBut | tert.-Butylester |
| OMe | Methylester |
| OEt | Ethylester |
| POA | Phenoxyacetyl |
| TFA | Trifluoressigsäure |
| Trt | Trityl (Triphenylmethyl). |

Sofern die vorstehend genannten Aminosäuren in mehreren enantiomeren Formen auftreten können, so sind vor- und nachstehend, z.B. als Bestandteil der Verbindungen der Formel I, alle diese Formen und auch ihre Gemische (z. B. die DL-Formen) eingeschlossen, wobei der Dreibuchstaben-Code ohne Angabe der Stereochemie für die jeweilige L-Form steht.

Gegenstand der Erfindung ist ferner ein Verfahren zur Herstellung einer Verbindung der Formel I nach Anspruch 1 oder eines ihrer Salze, dadurch gekennzeichnet, daß man sie aus einem ihrer funktionellen Derivate durch Behandeln mit einem solvolysierenden oder hydrogenolysierenden Mittel in Freiheit setzt
oder daß man ein Peptid der Formel II

$$X\text{-}M\text{-}OH \hspace{12em} II$$

worin

M   einen Aminosäure- oder Peptidrest, ausgewählt aus einer Gruppe bestehend aus A, A-Cys($R^1$), Ala, Thr, Thr-Ala, Lys, Lys-Thr, Lys-Thr-Ala, Lys-Thr-Ala-Gly, Gly, Gly-Lys, Gly-Lys-Thr, Gly-Lys-Thr-Ala, Gly-Lys-Thr-Cys($R^1$), Asn, Asn-Gly, Asn-Gly-Lys, Lys-Ala, Lys-Ala-Ala, Asn-Gly-Lys-Thr, Asn-Gly-Lys-Thr-Ala, Cys, Cys-Asn, Cys-Asn-Gly, Arg, Arg-Thr, Arg-Thr-Ala, Ser, Cys-Asn-Gly-Lys, Cys-Asn-Gly-Lys-Thr, Cys-Asn-Gly-Lys-Thr-Ala, Ser-Ala, Tyr, Tyr-Cys, Tyr-Cys-Asn, Tyr-Cys-Asn-Gly, Tyr-Cys-Asn-Gly-Lys, Gln, Gln-Ser, Gln-Ser-Ala, Tyr-Cys-Asn-Gly-Lys-Thr, Tyr-Cys-Asn-Gly-Lys-Thr-Ala, Asp, Asp-Tyr, Asp-Tyr-Cys, Asp-Tyr-Cys-Asn, Asp-Tyr-Cys-Asn-Gly, Asp-Tyr-Cys-Asn-Gly-Lys, Ile, Ile-Ser, Ile-Ser-Ala, Asp-Tyr-Cys-Asn-Gly-Lys-Thr, Arg-Ser, Arg-Ser-Ala, Asp-Tyr-Cys-Asn-Gly-Lys-Thr-Ala, Asp-Asp, Asp-Asp-Tyr, Asp-Asp-Tyr-Cys, Asp-Asp-Tyr-Cys-Asn, Asp-Asp-Tyr-Cys-Asn-Gly, Asp-Asp-Tyr-Cys-Asn-Gly-Lys, Asp-Asp-Tyr-Cys-Asn-Gly-Lys-Thr, Asp-Asp-Tyr-Cys-Asn-Gly-Lys-Thr-Ala, Met, Met-Asp, Met-Asp-Asp, Met-Asp-Asp-Tyr, Met-Asp-Asp-Tyr-Cys, Met-Asp-Asp-Tyr-Cys-Asn, Met-Asp-Asp-Tyr-Cys-Asn-Gly, Met-Asp-Asp-Tyr-Cys-Asn-Gly-Lys, Met-Asp-Asp-Tyr-Cys-Asn-Gly-Lys-Thr, Met-Asp-Asp-Tyr-Cys-Asn-Gly-Lys-Thr-Ala, Asp-Met, Asp-Met-Asp, Asp-Met-Asp-Asp, Asp-Met-Asp-Asp-Tyr, Asp-Met-Asp-Asp-Tyr-Cys, Asp-Met-Asp-Asp-Tyr-Cys-Asn, Asp-Met-Asp-Asp-Tyr-Cys-Asn-Gly, Asp-Met-Asp-Asp-Tyr-Cys-Asn-Gly-Lys, Asp-Met-Asp-Asp-Tyr-Cys-Asn-Gly-Lys-Thr, Asp-Met-Asp-Asp-Tyr-Cys-Asn-Gly-Lys-Thr-Ala, A-Cys($R^1$)-Pro, A-Cys($R^1$)-Pro-Arg, A-Cys($R^1$)-Pro-Arg-Asn, A-Cys($R^1$)-Pro-Arg-Asn-Pro, A-Cys($R^1$)-Pro-Arg-Asn-Pro-His, A-Cys($R^1$)-Pro-Arg-Asn-Pro-His-Lys, A-Cys($R^1$)-Pro-Arg-Asn-Pro-His-Lys-Gly, A-Cys($R^1$)-Pro-Arg-Asn-Pro-His-Lys-Gly-Pro, A-Cys($R^1$)-Pro-Arg-Asn-Pro-His-Lys-Gly-Pro-Ala,

wobei A und $R^1$ den in Anspruch 1 angegebenen Definitionen entsprechen, bedeutet
und

X   die angegebene Bedeutung besitzt, aber ungleich Wasserstoff ist, sofern A und damit M fehlen, mit einer Aminoverbindung der Formel III

$$H\text{-}Q\text{-}Z \hspace{12em} (III),$$

worin

Z   die angegebene Bedeutung besitzt und

Q   einen Aminosäure- oder Peptidrest, ausgewählt aus einer Gruppe bestehend aus B, Cys($R^1$)-B, Arg-Asn, Arg-Asn-Pro, Asn-Pro, Arg-Asn-Pro-His, Asn-Pro-His, Pro-His, Arg-Asn-Pro-His-Lys, Asn-Pro-His-Lys, Pro-His-Lys, His-Lys, Arg-Asn-Pro-His-Lys-Gly, Asn-Pro-His-Lys-Gly, Pro-His-Lys-Gly, His-Lys-Gly, Lys-Gly, Arg-Asn-Pro-His-Lys-Gly-Pro, Asn-Pro-His-Lys-Gly-Pro, Pro-His-Lys-Gly-Pro, His-Lys-Gly-Pro, Lys-Gly-Pro, Gly-Pro, Arg-Asn-Pro-His-Lys-Gly-Pro-Ala, Asn-Pro-His-Lys-Gly-Pro-Ala, Pro-His-Lys-Gly-Pro-Ala, His-Lys-Gly-Pro-Ala, Lys-Gly-Pro-Ala, Gly-Pro-Ala, Pro-Ala, Arg-Asn-Pro-His-Lys-Gly-Pro-Ala-Thr, Asn-Pro-His-Lys-Gly-Pro-Ala-Thr, Pro-His-Lys-Gly-Pro-Ala-Thr, His-Lys-Gly-Pro-Ala-Thr, Lys-Gly-Pro-Ala-Thr, Gly-Pro-Ala-Thr, Pro-Ala-Thr, Ala-Thr, Gly-Asp-Cys($R^1$)-B, Thr-Gly-Asp-Cys($R^1$)-B, Asp-Cys($R^1$)-B, Ala-Asp-Cys($R^1$)-B, Thr-Ala-Asp-Cys($R^1$)-B, Lys-Thr-Ala-Asp-Cys($R^1$)-B, Gly-Lys-Thr-Ala-Asp-Cys($R^1$)-B, Asn-Gly-Lys-Thr-Ala-Asp-Cys($R^1$)-B, Asn-Cys($R^1$)-B, Ala-Asn-Cys($R^1$)-B, Thr-Ala-Asn-Cys($R^1$)-B, Cys-Asn-Gly-Lys-Thr-Ala-Asp-Cys($R^1$)-B, Cys-Asn-Gly-Lys-Thr-Ala-Asp-Cys($R^1$)-B, Ala-Ala-Asp-Cys($R^1$)-B, Ser-Ala-Asp-Cys($R^1$)-B, Tyr-Cys-Asn-Gly-Lys-Thr-Ala-Asp-Cys($R^1$)-B, Gly-Cys($R^1$)-B, Ala-Gly-Cys($R^1$)-B, Ser-Ala-Gly-Cys($R^1$)-B, Cys(Trt)-Asp-Cys($R^1$)-B, Thr-Cys(Trt)-Asp-Cys($R^1$)-B, Lys-Thr-Cys(Trt)-Asp-Cys($R^1$)-B, Asp-Tyr-Cys-Asn-Gly-Lys-Thr-Ala-Asp-Cys(Trt)-B, Asp-Asp-Tyr-Cys-Asn-Gly-Lys-Thr-Ala-Asp-Cys($R^1$)-B oder Met-Asp-Asp-Tyr-Cys-Asn-Gly-Lys-Thr-Ala-Asp-Cys(Trt)-B,

wobei $R^1$ die angegebene Bedeutung besitzt,
bedeutet,
umsetzt,

und/oder daß man eine freie Mercapto-, Hydroxy- oder Aminogruppe alkyliert
und/oder eine Verbindung der Formel I durch Behandeln mit einer Säure oder Base in eines ihrer Salze überführt.

Der Rest A bedeutet vorzugsweise Ac-Asp, Ala-Asp, Thr-Ala-Asp, Lys-Thr-Ala-Asp, Ac-Lys-Thr-Ala-Asp, Gly-Lys-Thr-Cys-Asp, Gly-Lys-Thr-Cys(Trt)-Asp, Gly-Lys-Thr-Ala-Asp, Lys-Thr-Ala-Asn, Lys-Thr-Gly-Asp, Lys-Ala-Ala-Asp, Arg-Thr-Ala-Asp, Gly-Ser-Ala-Asp, Ac-Gln-Ser-Ala-Asp, Ile-Ser-Ala-Gly oder Arg-Ser-Ala-Gly.

B ist vorzugsweise nicht vorhanden oder bedeutet besonders bevorzugt Ala, welches gegebenenfalls methyliert sein kann, Pro, Pro-Arg, Pro-Arg-Asn, Pro-Arg-Asn-Pro, Pro-Arg-Asn-Pro-His, Pro-Arg-Asn-Pro-His-Lys, Pro-Arg-Asn-Pro-His-Lys-Gly oder Pro-Arg-Asn-Pro-His-Lys-Gly-Pro-Ala-Thr, während X vorzugsweise H oder Acetyl und Z besonders bevorzugt OH oder $NH_2$ bedeuten.

$R^1$ steht besonders bevorzugt für einen Triphenylmethylrest, während $R^2$ vorzugsweise Methyl, ferner aber auch bevorzugt Ethyl, Propyl, Butyl oder tert.-Butyl bedeutet.

Der Rest Ac bedeutet vorzugsweise Acetyl, kann aber ferner auch für Formyl, Propionyl, Butyryl, Isobutyryl, Valeryl, Isovaleryl, Pivaloyl (Trimethylacetyl), weiterhin bevorzugt für gegebenenfalls ein- bis dreifach substituiertes Aroyl mit 7-11 C-Atomen, wobei als Substituenten vorzugsweise eine der folgenden Gruppen in Frage kommen: Alkyl, Alkoxy, Alkylthio, Alkylsulfinyl oder Alkylsulfonyl mit jeweils 1-3, vorzugsweise 1 oder 2 C-Atomen, Methylendioxy, ferner OH, F, Cl, Br, I, $NO_2$, $NH_2$, Alkylamino oder Dialkylamino mit jeweils 1-3, vorzugsweise 1 oder 2 C-Atomen in der Alkylgruppe. Einzelne bevorzugte Aroylreste sind Benzoyl, o-, m- oder p-Toluyl, o-, m- oder p-Methoxybenzoyl, 2,3-, 2,4-, 2,5-, 2,6-, 3,4-, oder 3,5-Dimethoxybenzoyl, 2,3,4-, 2,3,5-, 2,3,6-, 2,4,5-, 2,4,6- oder 3,4,5-Trimethoxybenzoyl, o-, m- oder p-Methylsulfonylbenzoyl, 2,3- oder 3,4-Methylendioxybenzoyl, 1-oder 2-Naphthoyl. Ac kann weiterhin für Aralkanoyl mit 1-10 C-Atomen wie z.B. Phenylacetyl, 2-oder 3-Phenylpropionyl, 2-, 3- oder 4-Phenylbutyryl oder 2- oder 3-Phenylisobutyryl stehen.

Dementsprechend sind Gegenstand der Erfindung insbesondere diejenigen Verbindungen der Formel I, in denen mindestens einer der genannten Reste eine der angegebenen, insbesondere der angegebenen bevorzugten Bedeutungen hat.

Einige bevorzugte Gruppen von Verbindungen können durch die folgenden Teilformeln Ia bis Id ausgedrückt werden, die der Formel I entsprechen und worin die nicht näher bezeichneten Reste und Parameter die bei der Formel I angegebene Bedeutung haben, worin jedoch

in Ia    Cys($R^1$) Cys(Trt) und B Pro bedeuten;

in Ib    Cys($R^1$) Cys (Trt) und B
         Pro-Arg-Asn-Pro-His-Lys-Gly-Pro-Ala-Thr;

in Ic    Cys($R^1$) Cys(Trt) und
         B Pro-Arg, Pro-Arg-Asn-Pro, Pro-Arg-Asn-Pro-His,
         Pro-Arg-Asn-Pro-His-Lys oder Pro-Arg-Asn-Pro-His-Lys-Gly;

in Id    Cys($R^1$) Cys(Trt) und
         A Ala-Asp oder Lys-Thr-Ala-Asp

bedeuten.

Eine weitere Gruppe von bevorzugten Verbindungen kann durch die Teilformeln Iaa bis Ida ausgedrückt werden, die sonst der Formel I bzw. den Formeln Ia bis Id entsprechen, worin aber zusätzlich X Wasserstoff und Z OH oder $NH_2$ bedeuten.

Die Verbindungen der Formel I und auch die Ausgangsstoffe zu ihrer Herstellung werden im übrigen nach bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z.B. solche, die der Formel I entsprechen, aber anstelle einer $NH_2$-Gruppe eine NHR'-Gruppe (worin R' eine Aminoschutzgruppe bedeutet, z.B. Fmoc, BOC oder CBZ) enthalten.

Ferner sind Ausgangsstoffe bevorzugt, die anstelle des H-Atoms einer Hydroxygruppe eine Hydroxyschutzgruppe tragen, z.B. solche, die der Formel I entsprechen, aber anstelle einer Hydroxyphenylgruppe eine R"O-phenylgruppe enthalten (worin R" eine Hydroxyschutzgruppe bedeutet).

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren und den vorliegenden Verbindungen in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2- Trichlorethoxycarbonyl, BOC, 2-Iodethoxycarbonyl; Aralkyloxycarbonyl wie CBZ ("Carbobenzoxy"), 4-Methoxybenzyloxycarbonyl, Fmoc; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl und Acetyl, wobei Benzyl und Acetyl besonders bevorzugt sind. Die COOH-Gruppen in Asparaginsäure und Glutaminsäure werden bevorzugt in Form ihrer tert.-Butylester geschützt (z.B. Asp (OBut)).

Die als Ausgangsstoffe zu verwendenden funktionellen Derivate der Verbindungen der Formel I können nach üblichen Methoden der Aminosäure- und Peptidsynthese hergestellt werden, wie sie z.B. in den genannten Standardwerken und Patentanmeldungen beschrieben sind, z.B. auch nach der Festphasenmethode nach Merrifield (B.F. Gysin u. R.B. Merrifield, J. Am. Chem. Soc. 94, 3102 ff. (1972)) oder neueren an sich bekannten, daraus abgeleiteten, modernen Varianten.

Das In-Freiheit-Setzen der Verbindungen der Formel I aus ihren funktionellen Derivaten gelingt - je nach der benutzten Schutzgruppe - z.B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Isopropanol, sek.- oder tert.-Butanol, in Einzelfällen auch Methanol oder Ethanol sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, But, OBut, Trt und Mtr können z.B. bevorzugt mit TFA in Dichlormethan oder mit etwa 3 bis 5 n HCl in Dioxan bei 0-30° abgespalten werden, dabei können Hilfsreagenzien wie Anisol, Thiophenol oder Thioanisol die Reaktion günstig beeinflussen. Die Abspaltung der Fmoc-Gruppe erfolgt beispielsweise mit einer etwa 5- bis 50%igen Lösung von Dimethylamin, Diethylamin, Morpholin oder Piperidin in DMF bei 0-30°. Es ist dabei möglich, die Trt-Gruppe gezielt von Aminosäureresten abzuspalten, an die sie über Sauerstoff gebunden ist, während eine über Schwefel gebundene Trt-Gruppe im Molekül verbleibt. Ebenso kann ein Trt-Rest nachträglich eingeführt werden, indem bevorzugt eine Bindung zum nucleophilen Schwefel erfolgt, OH-Gruppen in der Seitenkette jedoch nicht substituiert werden.

Hydrogenolytisch entfernbare Schutzgruppen (z.B. CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol, Ether wie THF, Carbonsäuren wie Essigsäure, Wasser oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5 bis 10%igem Pd-C in Methanol oder mit Ammoniumformiat (anstelle von H2) an Pd-C in Wasser/DMF bei 20-30°.

Verbindungen der Formel I können auch durch Umsetzung einer Verbindung der Formel II mit einer Aminoverbindung der Formel III unter für Peptidsynthesen an sich bekannten, kondensierenden Bedingungen, wie sie z.B. in Houben-Weyl, l.c., Bd. 15/II, S. 1-806(1974) beschrieben sind, erhalten werden.

Die Reaktion gelingt vorzugsweise in Gegenwart eines Dehydratisierungsmittels, z.B. eines Carbodiimids wie DCCI oder EDCI, ferner Propanphosphonsäureanhydrid (vgl. Angew. Chem. 92, 129 (1980)), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, oder in Gemischen dieser Lösungsmittel, bei Temperaturen zwischen etwa -10 und 40, vorzugsweise zwischen 0 und 30°.

Anstelle von II können auch geeignete reaktionsfähige Derivate dieser Stoffe in die Reaktion eingesetzt werden, z.B. solche, in denen reaktive Gruppen intermediär durch Schutzgruppen blockiert sind. Die Aminosäurederivate II können z.B. in Form ihrer aktivierten Ester verwendet werden, die zweckmäßig in situ gebildet werden, z.B. durch Zusatz von HOBt oder N-Hydroxysuccinimid. Sie können aber auch in Form ihrer gemischten Anhydride eingesetzt werden, welche mit Carbonsäurehalogeniden wie Pivaloylchlorid oder Iso-butyloxycarbonylchlorid darstellbar sind.

Die Ausgangsstoffe der Formel II sind in der Regel neu. Sie können nach bekannten Methoden, z.B. den oben angegebenen Methoden der Peptidsynthese und der Abspaltung von Schutzgruppen, hergestellt werden.

In der Regel synthetisiert man zunächst geschützte Peptidester der Formel R'-M'-OR", z.B. BOC-M-OMe oder Fmoc-M-OBut. Diese werden zu Säuren der Formel R'-M-OH, z.B. BOC-M-OH oder Fmoc-M-OH verseift und dann mit einer Verbindung der Formel III, die gegebenenfalls ebenfalls durch entsprechende Schutzgruppen an Positionen, die der Reaktion nicht zugänglich sein sollen, versehen ist, kondensiert.

Im Falle von Verbindungen der Formel III synthetisiert man ebenfalls Peptidester der Formel R'-Q-Z'-R", wie z.B. BOC-Q-Z'-OMe oder Fmoc-Q-Z'-OMe, wobei Z' -NH- oder -O- bedeutet und spaltet dann, ehe man die Kondensation zur Herstellung von Verbindungen der Formel I durchführt, die Schutzgruppe R' auf bekannte Weise, z.B. Fmoc durch Behandlung mit einer Piperidin/DMF-Lösung, ab.

Besonders vorteilhaft können die neueren Methoden der Peptidsynthese nach modifizierten Merrifield-Techniken und unter Verwendung von Peptidsynthesegeräten, wie sie z.B. in Peptides, Proc. 8th Am. Pept. Symp., Eds. V. Hruby und D.H. Rich, Pierce Comp. III, p. 73-77 (1983) von A. Jonczyk und J. Meinenhofer (Fmoc-Strategie) beschrieben sind, oder die in Angew. Chem. 104, 375-391 (1992) dargestellten Techniken, Verwendung finden. Derartige Methoden sind an sich bekannt.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Andererseits kann eine Säure der Formel I durch Umsetzung mit einer Base in eines ihrer physiologisch unbedenklichen Metall- oder Ammoniumsalze übergeführt werden. Als Salze kommen dabei insbesondere die Natrium-, Kalium-, Magnesium-, Calcium- und Ammoniumsalze in Betracht, ferner substituierte Ammoniumsalze, z.B. die Dimethyl-, Diethyl- oder Diisopropylammoniumsalze, Monoethanol-, Diethanol- oder Triethanolammoniumsalze, Cyclohexyl-, Dicyclohexylammoniumsalze, Dibenzylethylendiammoniumsalze, weiterhin z.B. Salze mit N-Methyl-D-glucamin oder mit Arginin oder Lysin.

Die neuen Verbindungen der Formel I können ferner als Integrinliganden zur Herstellung von Säulen für die Affinitätschromatographie zur Reindarstellung von Integrinen verwendet werden.

Der Ligand, d.h. ein Peptidderivat der Formel I, wird dabei über Ankerfunktionen an einen polymeren Träger kovalent gekuppelt.

Als polymere Trägermaterialien eignen sich die an sich in der Peptidchemie bekannten polymeren festen Phasen mit vorzugsweise hydrophilen Eigenschaften, beispielsweise quervernetzte Polyzucker, wie Zellulose, Sepharose oder Sephadex®, Acrylamide, Polymere auf Polyethylenglykolbasis oder Tentakelpolymere®.

Als Ankerfunktionen, die mit den polymeren Trägern verknüpft sind, eignen sich vorzugsweise lineare Alkylenketten mit 2-12 C-Atomen, die mit einem Ende direkt an das Polymer gebunden sind und am anderen Ende eine funktionelle Gruppe, wie z.B. Hydroxy, Amino, Mercapto, Maleinimido oder -COOH aufweisen und dazu geeignet sind, mit dem C- oder N-terminalen Abschnitt des jeweiligen Peptids verknüpft zu werden.

Dabei ist es möglich, daß das Peptid direkt oder gegebenfalls über eine zweite Ankerfunktion mit dem Anker des Polymers verbunden ist. Ferner ist es möglich, daß Peptide, die Aminosäurereste mit funktionalisierten Seitenketten enthalten, über diese mit der Ankerfunktion des Polymers verbunden werden.

Darüber hinaus können bestimmte Aminosäurereste, die Bestandteil der Peptide der Formel I sind, in ihren Seitenketten derart modifiziert werden, daß sie zur Verankerung über z.B. SH-, OH-, $NH_2$- oder COOH-Gruppen mit dem Anker des Polymers zur Verfügung stehen.

Möglich sind hierbei ungewöhnliche Aminosäuren, wie z.B. Phenylalaninderivate, die in 4-Position des Phenylrings eine Mercapto-, Hydroxy-, Amino- oder Carboxyalkylkette tragen, wobei die funktionelle Gruppe sich am Ende der Kette befindet.

Beispiele für Aminosäurereste, deren Seitenkette direkt als Ankerfunktion dienen kann, sind z.B. Lys, Orn, Arg, Asp, Asn, Glu, Gln, Ser, Thr, Cys oder Tyr.

Beispiele für N-terminale Anker sind Reste wie z.B. $-CO-C_nH_{2n}-NH_2$, $-CO-C_nH_{2n}-OH$, $-CO-C_nH_{2n}-SH$ oder $-CO-C_nH_{2n}-COOH$ mit n = 2-12, wobei die Länge der Alkylenkette nicht kritisch ist und diese gegebenenfalls auch z.B. durch entsprechende Aryl- oder Alkylarylreste teilweise oder vollständig ersetzt werden kann.

C-terminale Anker können beispielsweise $-O-C_nH_{2n}-SH$, $-O-C_nH_{2n}-OH$, $-O-C_nH_{2n}-NH_2$, $-O-C_nH_{2n}-COOH$, $-NH-C_nH_{2n}-SH$, $-NH-C_nH_{2n}-OH$, $-NH-C_nH_{2n}-NH_2$ oder $-NH-C_nH_{2n}-COOH$ sein, wobei für n sowie für die Alkylenkette das bereits im vorhergehenden Abschnitt gesagte gilt.

Die N- und C-terminalen Anker können auch als Ankerbaustein für eine bereits funktionalisierte Seitenkette eines Aminosäurerests dienen. Es kommen hier beispielsweise Aminosäurereste wie $Lys(CO-C_5H_{10}-NH_2)$, $Asp(NH-C_3H_6-COOH)$ oder $Cys(C_3H_6-NH_2)$ in Frage, wobei der Anker immer an die funktionelle Gruppe der Seitenkette gebunden ist.

Die Herstellung der Materialien für die Affinitätschromatographie erfolgt unter Bedingungen wie sie für die Kondensation von Aminosäuren üblich und an sich bekannt sind und bereits im Abschnitt zur Herstellung der Verbindungen der Formel I geschildert wurden, bzw. in Pierce, Immuno Technology Catalog & Handbook (1990) beschrieben sind.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale), parenterale (z.B. intravenöse Injektion) oder lokale (z.B. topische, dermale, ophthalmische oder nasale) Applikation oder für eine Applikation in Form eines Inhalations-Sprays eignen und mit den neuen Verbindungen nicht reagieren, beispielsweise Wasser oder wässerige isotonische Kochsalzlösung, niedere Alkohole, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose, Vaseline. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Zur topischen Anwendung eignen sich z.B. Lösungen, die in Form von Augentropfen verwendet werden können, ferner z.B. Suspensionen, Emulsionen, Cremes, Salben oder Komprimate. Für die Applikation als Inhalations-Spray können Sprays verwendet werden, die den Wirkstoff entweder gelöst oder suspendiert in einem Treibgas oder Treibgasgemisch (z.B. $CO_2$ oder Fluorchlorkohlenwasserstoffen bzw. geeignete Ersatzstoffe) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die Injektionen können dabei als Bolus oder als kontinuierliche Infusion (z. B. intravenös, intramusculär, subcutan oder intrathecal) gegeben werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen können in der Regel in Analogie zu anderen bekannten, im Handel befindlichen Peptiden, insbesondere aber in Analogie zu den in der US-A-4 472 305 beschriebenen Verbindungen verabreicht werden, vorzugsweise in Dosierungen zwischen etwa 0,05 und 500, insbesondere zwischen 0,5 und 100 mg pro Dosierungseinheit. Die tägliche Dosierung liegt vorzugsweise zwischen etwa 0,01 und 2 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, neutralisiert, extrahiert mit Dichlormethan, trennt ab, trocknet die organische Phase über Natriumsulfat, filtriert, dampft ein und reinigt durch Chromatographie an Kieselgel und/oder Kristallisation. "Übliche Reinigung" bedeutet, daß man das Peptid aus TFA/$CH_2Cl_2$ mit Diethylether fällt und anschließend eine Gelfiltration in wässerigen Puffern und/oder eine Ionenaustauschchromatographie durchführt. RZ = Retentionszeit (Minuten) bei HPLC an Lichrosorb RP® select B(250-4,7 mm)-Säule, Laufmittel: 0,3 % TFA in Wasser; Isopropanolgradient von 0-80 Vol% in 50 Min. bei 1 ml/Min. Fluß und Detektion bei 215 nm. M+ = Molekular-Peak im Massenspektrum, erhalten nach der "Fast Atom Bombardment"-Methode (FAB), steht in der Regei für M+ + H, also die um 1 Masseneinheit erhöhte Masse der jeweiligen Verbindung. DMPP-Harz steht für 4-(2',4'-Dimethoxyphenyl-hydroxymethyl)-phenoxy-Harz, ein supersäurelabiles Resin, welches die Synthese von seitenkettengeschützten Peptiden erlaubt.

## Beispiel 1

0,6 g Fmoc-Pro-OH werden in 100 ml Dichlormethan gelöst, mit 1,2 Äquivalenten Wang-Harz (p-Benzyloxybenzylalkohol-Harz) versetzt und 12 Stunden bei Raumtemperatur gerührt. Nach Entfernung des Lösungsmittels erhält man Fmoc-Pro-Wang-Harz. Im Peptid-Synthesizer kondensiert man Fmoc-Cys(Trt)-OH mit H-Pro-Wang-Harz [Freisetzung aus Fmoc-Pro-Wang-Harz mit Piperidin/DMF (20%ig)], indem man den dreifachen Überschuß des geschützten Cysteins einsetzt. Die Kupplung wird in DCCI/HOBt bei Raumtemperatur durchgeführt. Man erhält Fmoc-Cys(Trt)-Pro-Wang-Harz. Anschließende erneute Behandlung mit Piperidin/DMF (20%ig) liefert H-Cys(Trt)-Pro-Wang-Harz.

## Beispiel 2

Analog Beispiel 1 erhält man ausgehend von Fmoc-Gly-DMPP-Harz durch Kondensation mit Fmoc-Ala-OH, Fmoc-Ser(But)-OH und Fmoc-Ile-OH in der genannten Reihenfolge im Peptid-Synthesizer (continuous flow Prinzip) nach Durchführung der folgenden Schritte:

- Freisetzung von H-Gly-DMPP-Harz mit Piperidin/DMF (20%ig)
- Waschen mit Dimethylacetamid (DMA)
- Umsetzung mit Fmoc-Ala-OH in DCCI/HOBt bei Raumtemperatur
- Waschen und Behandlung mit Piperidin/DMF (20%ig)
- Kupplung des entstandenen H-Ala-Gly-DMPP-Harz mit Fmoc-Ser(But)-OH
- Waschen und Behandlung des entstandenen Fmoc-Ser(But)-Ala-Gly-DMPP-Harz mit $CF_3SO_3H/CH_2Cl_2/H_2O$

Fmoc-Ser(But)-Ala-Gly-OH.

## Beispiel 3

Analog Beispiel 2 erhält man ausgehend von Fmoc-Asn(Trt)-DMPP-Harz nach Durchführung der entsprechenden Reaktionsschritte

durch Kupplung mit Fmoc-Ala-OH, Fmoc-Thr(But)-OH und Fmoc-Lys(Boc)-OH in der genannten Reihenfolge das Fmoc-Lys(Boc)-Thr(But)-Ala-Asn(Trt)-OH.

## Beispiel 4

Analog Beispiel 2 erhält man ausgehend von Fmoc-Asp(OBut)-DMPP-Harz nach Durchführung der entsprechenden Reaktionsschritte

durch Kupplung mit Fmoc-Gly-OH, Fmoc-Thr(But)-OH und Fmoc-Lys(Boc)-OH in der genannten Reihenfolge das Fmoc-Lys(Boc)-Thr(But)-Gly-Asp(OBut)-OH;

durch Kupplung mit Fmoc-Ala-OH, Fmoc-Ala-OH und Fmoc-Lys(Boc)-OH in der genannten Reihenfolge das Fmoc-Lys(Boc)-Ala-Ala-Asp(OBut)-OH;

durch Kupplung mit Fmoc-Ala-OH, Fmoc-Thr(But)-OH und Fmoc-Arg(Mtr)-OH in der genannten Reihenfolge das Fmoc-Arg(Mtr)-Thr(But)-Ala-Asp(OBut)-OH;

durch Kupplung mit Fmoc-Ala-OH und Fmoc-Ser(But)-OH in der genannten Reihenfolge das Fmoc-Ser(But)-Ala-Asp(OBut)-OH;

durch Kupplung mit Fmoc-Ala-OH, Fmoc-Thr(But)-OH und Fmoc-Lys(Boc)-OH in der genannten Reihenfolge das Fmoc-Lys(Boc)-Thr(But)-Ala-Asp(OBut)-OH;

durch Kupplung mit Fmoc-Ala-OH, Fmoc-Ser(But)-OH und Fmoc-Gln(Trt)-OH in der genannten Reihenfolge das Fmoc-Gln(Trt)-Ser(But)-Ala-Asp(OBut)-OH.

**Beispiel 5**

0,4 g H-Cys(Trt)-Pro-Wang-Harz werden im Peptid-Synthesizer (continuous flow Prinzip) mit Fmoc-Lys(Boc)-Thr(But)-Ala-Asn(Trt)-OH kondensiert, indem man den dreifachen Überschuß des Fmoc-Peptides einsetzt. Die Kupplung wird in DCCI/HOBt bei Raumtemperatur durchgeführt Man erhält Fmoc-Lys(Boc)-Thr(But)-Ala-Asn(Trt)-Cys(Trt)-Pro-Wang-Harz. Anschließende Behandlung mit TFA/CH$_2$Cl$_2$ und nachfolgende Abspaltung der Fmoc-Gruppe mit Piperidin/DMF (20%ig) liefert H-Lys-Thr-Ala-Asn-Cys(Trt)-Pro-OH.

Analog erhält man durch Kondensation von H-Cys(Trt)-Pro-Wang-Harz

mit Fmoc-Lys(Boc)-Thr(But)-Ala-Asp(Trt)-OH:
H-Lys-Thr-Ala-Asp-Cys(Trt)-Pro-OH; RZ = 28.9; M$^+$ = 876;
mit Fmoc-Lys(Boc)-Ala-Ala-Asp(Trt)-OH:
H-Lys-Ala-Ala-Asp-Cys(Trt)-Pro-OH;
mit Fmoc-Arg(Mtr)-Thr(But)-Ala-Asp(Trt)-OH:
H-Arg-Thr-Ala-Asp-Cys(Trt)-Pro-OH;
mit Fmoc-Ser(But)-Ala-Asp(Trt)-OH:
H-Ser-Ala-Asp-Cys(Trt)-Pro-OH;
mit Fmoc-Gln(Trt)-Ser(But)-Ala-Asp(Trt)-OH:
H-Gln-Ser-Ala-Asp-Cys(Trt)-Pro-OH;
mit Fmoc-Glp-Ser(But)-Ala-Asp(Trt)-OH:
H-Glp-Ser-Ala-Asp-Cys(Trt)-Pro-OH;
mit Fmoc-Lys(Boc)-Thr(But)-Ala-Asp(Trt)-OH:
H-Lys-Thr-Ala-Asp-Cys(Trt)-Pro-OH;
mit Fmoc-Ile-Ser(But)-Ala-Gly-OH:
H-Ile-Ser-Ala-Gly-Cys(Trt)-Pro-OH;
mit Fmoc-Arg(Mtr)-Ser(But)-Ala-Gly-OH:
H-Arg-Ser-Ala-Gly-Cys(Trt)-Pro-OH.
mit Fmoc-Lys(Boc)-Gly-Gly-Asp(Trt)-OH:
H-Lys-Gly-Gly-Asp-Cys(Trt)-Pro-OH.

**Beispiel 6**

Analog Beispiel 5 erhält man ausgehend von H-Cys(Trt)-Wang-Harz durch Kondensation mit Fmoc-Lys(Boc)-Thr(But)-Ala-Asp(OBut)-OH im Peptid-Synthesizer Fmoc-Lys(Boc)-Thr(But)-Ala-Asp(OBut)-Cys(Trt)-Wang-Harz, woraus nach Behandlung mit TFA/CH$_2$Cl$_2$, nachfolgender Abspaltung der Fmoc-Gruppe mit Piperidin/DMF (20%ig) sowie üblicher Reinigung H-Lys-Thr-Ala-Asp-Cys(Trt)-OH; RZ = 28.8; M+ = 779 erhalten wird.

**Beispiel 7**

1,2g BOC-Thr(But)-Ala-Asp(OBut)-Cys-Pro-Arg(Mtr)-OH werden in einer Mischung aus 150 ml Dichlormethan und 20 ml DMF gelöst, auf 0° abgekühlt und anschließend mit 0,5 g DCCI, 0,3 g HOBt, 0,23 ml N-Methylmorpholin und einem Äquivalent H-Asn(Trt)-Pro-His(Trt)-Lys(Boc)-Gly-Pro-Ala-Thr(But)-OMe [beide Peptide sind nach Methoden der modifizierten Merrifield-Technik erhältlich] versetzt. Es wird 20 Stunden bei 0° und 6 Stunden bei Raumtemperatur gerührt. Man engt die Reaktionsmischung ein, behandelt sie mit einem Ionenaustauscher und gibt sie in eine wäßrige NaHCO3-Lösung. Das ausfallende Produkt wird abgesaugt und mit Wasser gewaschen. Nach Kristallisation aus Ethylacetat/Petrolether erhält man BOC-Thr(But)-Ala-Asp(OB ut)-Cys-Pro-Arg(Mtr)-Asn(Trt)-Pro-His(Trt)-Lys(Boc)-Gly-Pro-Ala-Thr(But)-OMe.

Analog erhält man durch Kondensation

von BOC-Gly-Lys(Boc)-Thr(But)-Cys(Trt)-Asp(OBut)-OH mit H-Cys(Trt)-Pro-Arg(Mtr)-Asn(Trt)-Pro-His(Trt)-Lys(Boc)-Gly-Pro-Ala-Thr(But)-OMe:

BOC-Gly-Lys(Boc)-Thr(But)-Cys(Trt)-Asp(OBut)-Cys(Trt)-Pro-Arg(Mtr)-Asn(Trt)-Pro-His(Trt)-Lys(Boc)-Gly-Pro-Ala-Thr(But)-OMe;

von BOC-Lys(Boc)-Thr(But)-Ala-Asp(OBut)-Cys(Trt)-OH mit H-Pro-Arg(Mtr)-Asn(Trt)-Pro-His(Trt)-Lys(Boc)-Gly-OMe:

BOC-Lys(Boc)-Thr(But)-Ala-Asp(OBut)-Cys(Trt)- Pro-Arg(Mtr)-Asn(Trt)-Pro-His(Trt)-Lys(Boc)-Gly-OMe.

**Beispiel 8**

0,3 g BOC-Thr(But)-Ala-Asp(OBut)-Cys-Pro-Arg(Mtr)-Asn(Trt)-Pro-His(Trt)-Lys(Boc)-Gly-Pro-Ala-Thr(But)-OMe werden in 30 ml Methanol gelöst, mit 1,5 ml 2 N NaOH-Lösung versetzt und 4 Stunden bei 25° gerührt. Nach Entfernung des Lösungsmittels wird der Rückstand in Wasser aufgenommen, der pH-Wert durch Zugabe von verdünnter HCl auf 3 eingestellt und mit Ethylacetat extrahiert. Der Extrakt wird über $Na_2SO_4$ getrocknet. Nach Entfernung des Lösungsmittels erhält man BOC-Thr(But)-Ala-Asp(OBut)-Cys-Pro-Arg(Mtr)-Asn(Trt)-Pro-His(Trt)-Lys(Boc)-Gly-Pro-Ala-Thr(But)-OH, welches man in 20 ml 2 N HCl auf Dioxanbasis aufnimmt und 2 Stunden bei Raumtemperatur rührt. Die Reaktionsmischung wird zur Trockne eingeengt und der Rückstand in $TFA/CH_2Cl_2$ aufgenommen, mit $Et_2O$ gefällt und durch RP-HPLC gereinigt. Man erhält H-Thr-Ala-Asp-Cys-Pro-Arg-Asn-Pro-His-Lys-Gly-Pro-Ala-Thr-OH; RZ = 12.6; $M^+$ = 1465.

Analog erhält man durch Entfernung der Schutzgruppen ausgehend von den Verbindungen aus Beispiel 7:

H-Gly-Lys-Thr-Cys-Asp-Cys-Pro-Arg-Asn-Pro-His-Lys-Gly-Pro-Ala-Thr-OH; RZ = 13.1; $M^+$ = 1681;

H-Lys-Thr-Ala-Asp-Cys-Pro-Arg-Asn-Pro-His-Lys-Gly-OH.

**Beispiel 9**

Analog Beispiel 1 erhält man ausgehend von H-Thr(But)-Wang-Harz durch Kondensation mit Fmoc-Ala-OH, Fmoc-Pro-OH, Fmoc-Gly-OH, Fmoc-Lys(Boc)-OH, Fmoc-His(Trt)-OH und Fmoc-Pro-OH in der genannten Reihenfolge im Peptid-Synthesizer (continuous flow Prinzip) nach wiederholter Durchführung der oben angegebenen Schritte das Fmoc-Pro-His(Trt)-Lys(Boc)-Gly-Pro-Ala-Thr(But)-Wang-Harz, woraus nach erneuter Behandlung mit Piperidin/DMF (20%ig) H-Pro-His(Trt)-Lys(Boc)-Gly-Pro-Ala-Thr(But)-Wang-Harz erhalten wird.

**Beispiel 10**

Analog Beispiel 1 erhält man ausgehend von H-Gly-Wang-Harz durch Kondensation mit Fmoc-Lys(Boc)-OH, Fmoc-His(Trt)-OH und Fmoc-Pro-OH in der genannten Reihenfolge im Peptid-Synthesizer (continuous flow Prinzip) nach wiederholter Durchführung der oben angegebenen Schritte, das Fmoc-Pro-His(Trt)-Lys(Boc)-Gly-Wang-Harz, woraus nach erneuter Behandlung mit Piperidin/DMF (20%ig) H-Pro-His(Trt)-Lys(Boc)-Gly-Wang-Harz erhalten wird.

**Beispiel 11**

Analog Beispiel 2 erhält man ausgehend von Fmoc-Asn(Trt)-Wang-Harz nach Durchführung der entsprechenden Reaktionsschritte

durch Kupplung mit Fmoc-Arg(Mtr)-OH, Fmoc-Pro-OH, Fmoc-Cys(Trt)-OH, Fmoc-Asp(OBut)-OH und Fmoc-Ala-OH in der genannten Reihenfolge

das Fmoc-Ala-Asp(OBut)-Cys(Trt)-Pro-Arg(Mtr)-Asn(Trt)-OH.

Analog erhält man ausgehend von Fmoc-Asn(Trt)-Wang-Harz

durch Kupplung mit Fmoc-Arg(Mtr)-OH, Fmoc-Pro-OH, Fmoc-Cys(Trt)-OH, Fmoc-Asp(OBut)-OH, Fmoc-Ala-OH, Fmoc-Thr(But)-OH und Fmoc-Lys(Boc)-OH in der genannten Reihenfolge das

Fmoc-Lys(Boc)-Thr(But)-Ala-Asp(OBut)-Cys(Trt)-Pro-Arg(Mtr)-Asn(Trt)-OH;

durch Kupplung mit Fmoc-Arg(Mtr)-OH, Fmoc-Pro-OH, Fmoc-Cys(Trt)-OH, Fmoc-Asp(OBut)-OH, Fmoc-Cys(Trt)-OH, Fmoc-Thr(But)-OH, Fmoc-Lys(Boc)-OH und Fmoc-Gly-OH in der genannten Reihenfolge das

Fmoc-Gly-Lys(Boc)-Thr(But)-Cys(Trt)-Asp(OBut)-Cys(Trt)-Pro-Arg(Mtr)-Asn(Trt)-OH;

durch Kupplung mit Fmoc-Arg(Mtr)-OH, Fmoc-Pro-OH, Fmoc-Cys(Trt)-OH, Fmoc-Asp(OBut)-OH und Fmoc-Ala-OH in der genannten Reihenfolge das
 Fmoc-Ala-Asp(OBut)-Cys(Trt)-Pro-Arg(Mtr)-Asn(Trt)-OH;

durch Kupplung mit Fmoc-Arg(Mtr)-OH, Fmoc-Pro-OH, Fmoc-Cys(Trt)-OH, Fmoc-Asp(OBut)-OH, Fmoc-Ala-OH, Fmoc-Thr(But)-OH, Fmoc-Lys(Boc)-OH und Fmoc-Gly-OH in der genannten Reihenfolge das
 Fmoc-Gly-Lys(Boc)-Thr(But)-Ala-Asp(OBut)-Cys(Trt)-Pro-Arg(Mtr)-Asn(Trt)-OH;

durch Kupplung mit Fmoc-Arg(Mtr)-OH, Fmoc-Pro-OH, Fmoc-Cys(Trt)-OH, Fmoc-Asp(OBut)-OH, Fmoc-Ala-OH und Fmoc-Thr(But)-OH in der genannten Reihenfolge das
 Fmoc-Thr(But)-Ala-Asp(OBut)-Cys(Trt)-Pw-Arg(Mtr)-Asn(Trt)-OH.

**Beispiel 12**

Analog Beispiel 5 erhält man durch Kondensation von H-Pro-His(Trt)-Lys(Boc)-Gly-Pro-Ala-Thr(But)-Wang-Harz mit Fmoc-Ala-Asp(OBut)-Cys(Trt)-Pro-Arg(Mtr)-Asn(Trt)-OH das Fmoc-Ala-Asp(OBut)-Cys(Trt)-Pro-Arg(Mtr)-Asn(Trt)-Pro-His(Trt)-Lys(Boc)-Gly-Pro-Ala-Thr(But)-Wang-Harz. Anschließende Behandlung mit $TFA/CH_2Cl_2$ und nach- folgende Abspaltung der Fmoc-Gruppe mit Piperidin/DMF (20%ig) liefert:
H-Ala-Asp-Cys(Trt)-Pro-Arg-Asn-Pro-His-Lys-Gly-Pro-Ala-Thr-OH; RZ = 25.6; $M^+$ = 1606.
Analog erhält man durch Kondensation

von H-Pro-His(Trt)-Lys(Boc)-Gly-Pro-Ala-Thr(But)-Wang-Harz mit Fmoc-Lys(Boc)-Thr(But)-Ala-Asp(OBut)-Cys (Trt)-Pro-Arg(Mtr)-Asn(Trt)-OH das
 H-Lys-Thr-Ala-Asp-Cys(Trt)-Pro-Arg-Asn-Pro-His-Lys-Gly-Pro-Ala-Thr-OH; RZ = 24.6; $M^+$ = 1835;

von H-Pro-His(Trt)-Lys(Boc)-Gly-Pro-Ala-Thr(But)-Wang-Harz mit Fmoc-Gly-Lys(Boc)-Thr(But)-Cys(Trt)-Asp (OBut)-Cys(Trt)-Pro-Arg(Mtr)-Asn(Trt)-OH das
 H-Gly-Lys-Thr-Cys(Trt)-Asp-Cys(Trt)-Pro-Arg-Asn-Pro-His-Lys-Gly-Pro-Ala-Thr-OH; RZ = 30.8; $M^+$ = 2167;

von H-Pro-His(Trt)-Lys(Boc)-Gly-Wang-Harz mit Fmoc-Gly-Lys(Boc)-Thr(But)-Ala-Asp(OBut)-Cys(Trt)-Pro-Arg (Mtr)-Asn(Trt)-OH das
 H-Gly-Lys-Thr-Ala-Asp-Cys(Trt)-Pro-Arg-Asn-Pro-His-Lys-Gly-OH;
 RZ = 24.5; $M^+$ = 1623;

von H-Pro-His(Trt)-Lys(Boc)-Gly-Pro-Ala-Thr(But)-Wang-Harz mit Fmoc-Thr(But)-Ala-Asp(OBut)-Cys(Trt)-Pro-Arg(Mtr)-Asn(Trt)-OH das
 H-Thr-Ala-Asp-Cys-Pro-Arg-Asn-Pro-His-Lys-Gly-Pro-Ala-Thr-OH;
 RZ = 24.9; $M^+$ = 1707.

**Beispiel 13**

Analog Beispiel 7 und 8 erhält man durch Kondensation und anschließende Verseifung sowie Abspaltung der BOC-Schutzgruppe ausgehend

von H-Pro-His(Trt)-Lys(Boc)-Gly-Pro-Ala-Thr(But)-OMe mit Boc-Ala-Asp(OBut)-Cys(Trt)-Pro-Arg(Mtr)-Asn(Trt)-OH das
 H-Ala-Asp-Cys-Pro-Arg-Asn-Pro-His-Lys-Gly-Pro-Ala-Thr-OH; RZ = 13.1; $M^+$ = 1362;

von H-Pro-His(Trt)-Lys(Boc)-Gly-OMe mit Boc-Ala-Asp(OBut)-Cys(Trt)-Pro-Arg(Mtr)-Asn(Trt)-OH das
 H-Ala-Asp-Cys-Pro-Arg-Asn-Pro--His-Lys-Gly-OH; RZ = 10.6; $M^+$ = 1094;

von H-Pro-His(Trt)-Lys(Boc)-Gly-OMe mit Boc-Gly-Lys(Boc)-Thr(But)-Ala-Asp(OBut)-Cys(Trt)-Pro-Arg(Mtr)-Asn (Trt)-OH das
 H-Gly-Lys-Thr-Ala-Asp-Cys-Pro-Arg-Asn-Pro-His-Lys-Gly-OH; RZ = 11.4; $M^+$ = 1380;

von H-Pro-His(Trt)-Lys(Boc)-Gly-Pro-Ala-Thr(But)-OMe mit Boc-Lys(Boc)-Thr(But)-Ala-Asp(OBut)-Cys(Trt)-Pro-Arg(Mtr)-Asn(Trt)-OH das
H-Lys-Thr-Ala-Asp-Cys-Pro-Arg-Asn-Pro-His-Lys-Gly-Pro-Ala-Thr-OH; RZ = 12.7; M$^+$ = 1592.

## Beispiel 14

Analog Beispiel 2 erhält man ausgehend von Fmoc-Asp(OBut)-DMPP-Harz nach Durchführung der entsprechenden Reaktionsschritte

durch Kupplung mit Fmoc-Ala-OH, Fmoc-Thr(But)-OH und Fmoc-Lys(Boc)-OH in der genannten Reihenfolge das
Fmoc-Lys(Boc)-Thr(But)-Ala-Asp(OBut)-OH.

Analog erhält man ausgehend von Fmoc-Cys(Trt)-DMPP-Harz

durch Kupplung mit Fmoc-Asp(OBut)-OH in der genannten Reihenfolge das
Fmoc-Asp(OBut)-Cys(Trt)-OH;

durch Kupplung mit Fmoc-Asp(OBut)-OH und Fmoc-Ala-OH in der genannten Reihenfolge das
Fmoc-Ala-Asp(OBut)-Cys(Trt)-OH;

durch Kupplung mit Fmoc-Asp(OBut)-OH, Fmoc-Ala-OH und Fmoc-Thr(But)-OH in der genannten Reihenfolge das
Fmoc-Thr(But)-Ala-Asp(OBut)-Cys(Trt)-OH.

## Beispiel 15

Analog Beispiel 1 erhält man ausgehend von H-Pro-Wang-Harz durch Kondensation mit Fmoc-Asn(Trt)-OH, Fmoc-Arg(Mtr)-OH, Fmoc-Pro-OH und Fmoc-Cys(Trt)-OH in der genannten Reihenfolge im Peptid-Synthesizer (continuous flow Prinzip) nach wiederholter Durchführung der oben angegebenen Schritte, das Fmoc-Cys(Trt)-Pro-Arg(Mtr)-Asn(Trt)-Pro-Wang-Harz, woraus nach erneuter Behandlung mit Piperidin/DMF (20%ig) H-Cys(Trt)-Pro-Arg(Mtr)-Asn(Trt)-Pro-Wang-Harz erhalten wird.
Analog erhält man ausgehend

von Fmoc-Lys(Boc)-Wang-Harz durch Kupplung mit Fmoc-His(Trt)-OH, Fmoc-Pro-OH, Fmoc-Asn(Trt)-OH, Fmoc-Arg(Mtr)-OH, Fmoc-Pro-OH und Fmoc-Cys(Trt)-OH in der genannten Reihenfolge das
H-Cys(Trt)-Pro-Arg(Mtr)-Asn(Trt)-Pro-His(Trt)-Lys(Boc)-Wang-Harz;

von Fmoc-His(Trt)-Wang-Harz durch Kupplung mit Fmoc-Pro-OH, Fmoc-Asn(Trt)-OH, Fmoc-Arg(Mtr)-OH, Fmoc-Pro-OH und Fmoc-Cys(Trt)-OH in der genannten Reihenfolge das
H-Cys(Trt)-Pro-Arg(Mtr)-Asn(Trt)-Pro-His(Trt)-Wang-Harz;

von Fmoc-Gly-Wang-Harz durch Kupplung mit Fmoc-Lys(Boc)-OH, Fmoc-His(Trt)-OH, Fmoc-Pro-OH, Fmoc-Asn(Trt)-OH, Fmoc-Arg(Mtr)-OH, Fmoc-Pro-OH und Fmoc-Cys(Trt)-OH in der genannten Reihenfolge das
H-Cys(Trt)-Pro-Arg(Mtr)-Asn(Trt)-Pro-His(Trt)-Lys(Boc)-Gly-Wang-Harz;

von Fmoc-Arg(Mtr)-Wang-Harz durch Kupplung mit Fmoc-Pro-OH und Fmoc-Cys(Trt)-OH in der genannten Reihenfolge das
H-Cys(Trt)-Pro-Arg(Mtr)-Wang-Harz;

von Fmoc-Ala-Wang-Harz durch Kupplung mit Fmoc-Cys(Trt)-OH das
H-Cys(Trt)-Ala-Wang-Harz.

## Beispiel 16

Analog Beispiel 5 erhält man durch Kondensation von Fmoc-Lys(Boc)-Thr(B ut)-Ala-Asp(OB ut)-OH mit H-Cys(Trt)-Pro-Arg(Mtr)-Asn(Trt)-Pro-Wang-Harz das Fmoc-Lys(Boc)-Thr(B ut)-Ala-Asp(OBut)-Cys(Trt)-Pro-Arg(Mtr)-Asn(Trt)-Pro-Wang-Harz. Anschließende Behandlung mit TFA/CH$_2$Cl$_2$ und nachfolgende Abspaltung der Fmoc-Gruppe mit Piperidin/ DMF (20%ig) liefert:
H-Lys-Thr-Ala-Asp-Cys(Trt)-Pro-Arg-Asn-Pro-OH; RZ = 25.4; M$^+$ = 1243.
Analog erhält man durch Kondensation

von Fmoc-Lys(Boc)-Thr(But)-Ala-Asp(OBut)-OH mit H-Cys(Trt)-Pro-Arg(Mtr)-Asn(Trt)-Pro-His(Trt)-Lys(Boc)-Wang-Harz das

H-Lys-Thr-Ala-Asp-Cys(Trt)-Pro-Arg-Asn-Pro-His-Lys-OH; RZ = 23.6; $M^+$ = 1509;

von Fmoc-Lys(Boc)-Thr(But)-Ala-Asp(OBut)-OH mit H-Cys(Trt)-Pro-Arg(Mtr)-Asn(Tn)-Pro-His(Tn)-Wang-Harz das

H-Lys-Thr-Ala-Asp-Cys(Trt)-Pro-Arg-Asn-Pro-His-OH; RZ = 24.3; $M^+$ = 1380;

von Fmoc-Lys(Boc)-Thr(B ut)-Ala-Asp(OBut)-OH mit H-Cys(Trt)-Pro-Arg(Mtr)-Asn(Trt)-Pro-His(Trt)-Lys(Boc)-Gly-Wang-Harz das

H-Lys-Thr-Ala-Asp-Cys(Trt)-Pro-Arg-Asn-Pro-His-Lys-Gly-OH; RZ = 23.7; $M^+$ = 1565;

von Fmoc-Lys(Boc)-Thr(But)-Ala-Asp(OBut)-OH mit H-Cys(Trt)-Pro-Arg(Mtr)-Wang-Harz das
H-Lys-Thr-Ala-Asp-Cys(Trt)-Pro-Arg-OH; RZ = 25.7; $M^+$ = 1032;

von Fmoc-Asp(OBut)-Cys(Trt)-OH mit H-Pro-Arg(Mtr)-Asn(Trt)-Pro-His(Trt)-Lys(Boc)-Wang-Harz das
H-Asp-Cys(Trt)-Pro-Arg-Asn-Pro-His-Lys-OH;

von Fmoc-Ala-Asp(OBut)-Cys(Trt)-OH mit H-Pro-Arg(Mtr)-Wang-Harz das
H-Ala-Asp-Cys(Trt)-Pro-Arg-OH; RZ = 27.4; $M^+$ = 803;

von Fmoc-Thr(But)-Ala-Asp(OBut)-Cys(Trt)-OH mit H-Pro-Arg(Mtr)-Wang-Harz das
H-Thr-Ala-Asp-Cys(Trt)-Pro-Arg-OH; RZ = 27.3; $M^+$ = 904;

von Fmoc-Lys(Boc)-Thr(But)-Ala-Asp(OBut)-OH mit H-Cys(Trt)-Pro-Wang-Harz das
H-Lys-Thr-Ala-Asp-Cys(Trt)-Pro-OH.

## Beispiel 17

0,9 g H-Cys(Trt)-Pro-Arg-Asn-Pro-His-Lys(BOC)-DMPP-Harz OC)-DMPP-Harz [Herstellung nach Bsp. 1] werden in 100 ml Dichlormethan gelöst, analog Beispiel 2 mit $H_3$-CO-Asp-OH kondensiert und aufgearbeitet. Man erhält nach Wiedereinführung der Trt-Gruppe mit Triphenylmethanol $H_3$C-CO-Asp-Cys(Trt)-Pro-Arg-Asn-Pro-His-Lys-OH; RZ = 26.0; $M^+$ = 1250.
Analog erhält man ausgehend

von H-Thr-Ala-Asp-Cys(Trt)-Pro-OH mit $H_3$C-CO-Lys(BOC)-OH
$H_3$C-CO-Lys-Thr-Ala-Asp-Cys(Trt)-Pro-OH;

von H-Ser-Ala-Asp-Cys(Trt)-Pro-OH mit $H_3$CO-Gln(Trt)-OH
$H_3$C-CO-Gln-Ser-Ala-Asp-Cys(Trt)-Pro-OH.

## Beispiel 18

0,7 g Fmoc-Cys(Trt)-Pro-OH werden in 100 ml Dichlormethan gelöst, mit 1,4 Äquivalenten MBHA-Harz, 1,4 Äquivalenten HOBt und 1,4 Äquivalenten DCC versetzt und 24 Stunden bei Raumtemperatur gerührt. Nach Entfernung des Lösungsmittels erhält man Fmoc-Cys(Trt)-Pro-MBHA-Harz. Durch Behandlung (1 Std. bei Raumtemperatur) mit Piperidin/DMF (20%ig) erhält man daraus H-Cys(Trt)-Pro-MBHA-Harz, welches man anschließend mit Fmoc-Lys(Boc)-Thr(But)-Ala-Asp(OBut)-OH, durch Zugabe des dreifachen Überschusses dieser Verbindung, kuppelt. Die Kupplung wird in DCCI/HOBt bei Raumtemperatur durchgeführt. Man erhält Fmoc-Lys(Boc)-Thr(But)-Ala-Asp(OBut)-Cys(Trt)-Pro-MBHA-Harz. Anschließende erneute Behandlung mit Piperidin/DMF (20%ig) liefert H-Lys(Boc)-Thr(But)-Ala-Asp(OBut)-Cys(Trt)-Pro-MBHA-Harz.
Die erhaltene Verbindung wird in 20 ml TFA aufgenommen und 2 Stunden bei Raumtemperatur rührt. Man erhält nach üblicher Reinigung H-Lys-Thr-Ala-Asp-Cys(Trt)-Pro-NH$_2$.
Analog erhält man durch Umsetzung der freien Peptide mit MBHA-Harz und anschließender Abspaltung des Harzes die folgenden Peptidamide:

H-Lys-Thr-Ala-Asp-Cys(Trt)-Pro-Arg-Asn-Pro-His-Lys-NH$_2$;
H-Lys-Thr-Ala-Asp-Cys(Trt)-Pro-Arg-Asn-Pro-His-NH$_2$;

H-Lys-Thr-Ala-Asp-Cys(Trt)-Pro-Arg-Asn-Pro-His-Lys-Gly-NH$_2$;
H-Lys-Thr-Ala-Asp-Cys(Trt)-Pro-Arg-NH$_2$;
H-Asp-Cys(Trt)-Pro-Arg-Asn-Pro-His-Lys-NH$_2$;
H-Ala-Asp-Cys(Trt)-Pro-Arg-NH$_2$;
H-Thr-Ala-Asp-Cys(Trt)-Pro-Arg-NH$_2$;
H-Lys-Thr-Ala-Asp-Cys(Trt)-Pro-NH$_2$.

**Beispiel 19**

Analog Beispiel 17 erhält man durch Kondensation von H-Thr-Ala-Asp-Cys(Trt)-Pro-NH$_2$ mit H$_3$C-CO-Lys(BOC)-OH und anschließende Abspaltung der BOC-Gruppe das H$_3$C-CO-Lys-Thr-Ala-Asp-Cys(Trt)-Pro-NH$_2$.
Analog erhält man:

H$_3$C-CO-Lys-Thr-Ala-Asp-Cys(Trt)-Pro-Arg-Asn-Pro-His-Lys-NH$_2$;
H$_3$C-CO-Lys-Thr-Ala-Asp-Cys(Trt)-Pro-Arg-Asn-Pro-His-Lys-Gly-NH$_2$;
H$_3$C-CO-Lys-Thr-Ala-Asp-Cys(Trt)-Pro-Arg-NH$_2$;
H$_3$C-CO-Thr-Ala-Asp-Cys(Trt)-Pro-Arg-NH$_2$;
H$_3$C-CO-Lys-Thr-Ala-Asp-Cys(Trt)-Pro-NH$_2$.

**Beispiel 20**

Analog Beispiel 5 erhält man durch Kondensation von Fmoc-Lys(Boc)-Thr(But)-Ala-Asp(OBut)-OH mit H-Cys(Trt)-NMeAla-Wang-Harz das Fmoc-Lys(Boc)-Thr(But)-NMeAla-Wang-Harz. Anschließende Behandlung mit TFA/CH$_2$Cl$_2$ und nachfolgende Abspaltung der Fmoc-Gruppe mit Piperidin/DMF (20%ig) liefert:
H-Lys-Thr-Ala-Asp-Cys(Trt)-NMeAla-OH.

**Beispiel 21**

Man löst 0,2 g H-Lys-Thr-Ala-Asp-Cys-Pro-Arg-Asn-Pro-His-Lys-Gly-OH in 30 ml CH$_2$Cl$_2$ und 30 ml TFA und fügt bei Raumtemperatur 1,2 Äquivalente Triphenylmethylalkohol hinzu. Anschließend rührt man eine Stunde und fällt das entstandene Peptid nach Einengen durch Zugabe von Diethylether. Nach üblicher Reinigung erhält man
H-Lys-Thr-Ala-Asp-Cys(Trt)-Pro-Arg-Asn-Pro-His-Lys-Gly-OH;
RZ = 23.7; M$^+$ = 1565.
Analog erhält man durch Alkylierung von H-Lys-Thr-Ala-Asp-Cys-Pro-Arg-Asn-Pro-His-Lys-Gly-OH:

mit Methyliodid: H-Lys-Thr-Ala-Asp-Cys(Me)-Pro-Arg-Asn-Pro-His-Lys-Gly-OH; RZ = 9.8; M$^+$ = 1338;

mit Ethyliodid: H-Lys-Thr-Ala-Asp-Cys(Et)-Pro-Arg-Asn-Pro-His-Lys-Gly-OH; RZ = 10.4; M$^+$ = 1352;

mit Benzylchlorid: H-Lys-Thr-Ala-Asp-Cys(Bzl)-Pro-Arg-Asn-Pro-His-Lys-Gly-OH; RZ =13.8; M$^+$ = 1415;

mit tert.-Butylchlorid: H-Lys-Thr-Ala-Asp-Cys(tB u)-Pro-Arg-Asn-Pro-His-Lys-Gly-OH; RZ = 12.3; M$^+$ = 1379;

mit Diphenylmethylchlorid:
H-Lys-Thr-Ala-Asp-Cys(Dpm)-Pro-Arg-Asn-Pro-His-Lys-Gly-OH; RZ = 17.8; M$^+$ = 1489.

Die nachstehenden Beispiele betreffen pharmazeutische Zubereitungen.

**Beispiel A: Injektionsgläser**

Eine Lösung von 100 g eines Wirkstoffes der Formel I und 5 g Dinatriumhydrogenphosphat in 3 1 zweifach destilliertem Wasser wird mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

**Beispiel B: Suppositorien**

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

**Beispiel C: Lösung**

Es wird eine Lösung aus 1 g eines Wirkstoffes der Formel I, 9,38 g $NaH_2PO_4 \times 2 H_2O$, 28,48 g $Na_2HPO_4 \times 12 H_2O$ und 0,1 g Benzalkoniumchlorid in 940 ml zweifach destilliertem Wasser bereitet. Man stellt auf pH 6,8 ein, füllt auf 1 l auf und sterilisiert durch Bestrahlung. Diese Lösung kann in Form von Augentropfen verwendet werden.

**Beispiel D: Salbe**

Man mischt 500 mg eines Wirkstoffes der Formel I mit 99,5 g Vaseline unter aseptischen Bedingungen.

**Beispiel E: Tabletten**

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

**Beispiel F: Dragees**

Analog Beispiel E werden Tabletten gepreßt, die anschließend in üblicher Weise mit einem Überzug aus Saccharose, Kartoffelstärke, Talk, Tragant und Farbstoff überzogen werden.

**Beispiel G: Kapseln**

2 kg Wirkstoff der Formel I werden in üblicher Weise in Hartgelatinekapseln gefüllt, so daß jede Kapsel 20 mg des Wirkstoffs enthält.

**Beispiel H: Ampullen**

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

**Patentansprüche**

1. Lineare Peptide der Formel I mit einer Mindestlänge von 4 Aminosäureresten

$$X\text{-}A\text{-}Cys(R^1)\text{-}B\text{-}Z \hspace{6cm} I,$$

worin

X   H oder Ac,

A   fehlt, Asp oder ein Peptidfragment, ausgewählt aus einer Gruppe bestehend aus Ala-Asp, Thr-Ala-Asp, Lys-Thr-Ala-Asp, Lys-Thr-Ala-Asn, Lys-Thr-Gly-Asp, Lys-Ala-Ala-Asp, Arg-Thr-Ala-Asp, Ser-Ala-Asp, Gln-Ser-Ala-Asp, Gly-Lys-Thr-Ala-Asp, Asn-Gly-Lys-Thr-Ala-Asp, Ile-Ser-Ala-Gly, Arg-Ser-Ala-Gly, Cys-Asn-Gly-Lys-Thr-Ala-Asp; Tyr-Cys-Asn-Gly-Lys-Thr-Ala-Asp, Asp-Tyr-Cys-Asn-Gly-Lys-Thr-Ala-Asp, Gly-Lys-Thr-Cys-Asp, Asp-Asp-Tyr-Cys-Asn-Gly-Lys-Thr-Ala-Asp, Gly-Lys-Thr-Cys(Trt)-Asp, Met-Asp-Asp-Tyr-Cys-Asn-Gly-Lys-Thr-Ala-Asp, Asp-Met-Asp-Asp-Tyr-Cys-Asn-Gly-Lys-Thr-Ala-Asp,

B   fehlt, Ala, Arg, Asn, Asp, Cys, Gln, Glu, Gly, His, Ile, Leu, Lys, Met, Orn, Phe, 4-Hal-Phe, Pro, Ser, Thr, Trp, Tyr, Val oder ein N-methyliertes Derivat der genannten Aminosäurereste, oder ein Peptidfragment, ausgewählt aus einer Gruppe bestehend aus Pro-Arg, Pro-Arg-Asn, Pro-Arg-Asn-Pro, Pro-Arg-Asn-Pro-His, Pro-Arg-Asn-Pro-His-Lys, Pro-Arg-Asn-Pro-His-Lys-Gly, Pro-Arg-Asn-Pro-His-Lys-Gly-Pro, Pro-Arg-Asn-Pro-His-Lys-Gly-Pro-Ala, Pro-Arg-Asn-Pro-His-Lys-Gly-Pro-Ala-Thr,

wobei lediglich einer der Reste A oder B fehlen kann,

Z    OH, $OR^2$; $NH_2$, $NHR^2$ oder $NR_2^2$,

$R^1$    H, $R^2$, Trt, Dpm oder Bzl,

$R^2$    Alkyl mit 1-6 C-Atomen,

Hal    F, Cl, Br oder I und

Ac    Alkanoyl mit 1-10 C-Atomen, Aralkanoyl mit 8-10 C-Atomen oder Aroyl mit 7-11 C-Atomen

bedeuten,
sowie deren physiologisch unbedenkliche Salze.

**2.**    Ein Enantiomer oder ein Diastereomer einer Verbindung der Formel I gemäß Anspruch 1.

**3.**    (a) H-Ala-Asp-Cys(Trt)-Pro-Arg-Asn-Pro-His-Lys-Gly-Pro-Ala-Thr-OH;

    (b) H-Lys-Thr-Ala-Asp-Cys(Trt)-Pro-Arg-Asn-Pro-His-Lys-Gly-Pro-Ala-Thr-OH;

    (c) H-Gly-Lys-Thr-Ala-Asp-Cys(Trt)-Pro-Arg-Asn-Pro-His-Lys-Gly-OH;

    (d) H-Lys-Thr-Ala-Asp-Cys(Trt)-Pro-Arg-Asn-Pro-His-OH;

    (e) H-Lys-Thr-Ala-Asp-Cys(Trt)-Pro-Arg-Asn-Pro-His-Lys-Gly-OH;

    (f) H-Lys-Thr-Ala-Asp-Cys(Trt)-Pro-Arg-OH;

    (g) H-Lys-Thr-Ala-Asp-Cys(Trt)-Pro-OH

    (h) $H_3$C-CO-Asp-Cys(Trt)-Pro-Arg-Asn-Pro-His-Lys-OH.

**4.**    Verfahren zur Herstellung einer Verbindung der Formel I nach Anspruch 1 oder eines ihrer Salze, dadurch gekennzeichnet, daß man sie aus einem ihrer funktionellen Derivate durch Behandeln mit einem solvolysierenden oder hydrolysierenden Mittel in Freiheit setzt oder daß man eine Verbindung der Formel II

$$X\text{-}M\text{-}OH \qquad\qquad II,$$

worin

M  einen Aminosäure- oder Peptidrest, ausgewählt aus einer Gruppe bestehend aus A, A-Cys($R^1$), Ala, Thr, Thr-Ala, Lys, Lys-Thr, Lys-Thr-Ala, Gly, Lys-Thr-Ala-Gly, Gly-Lys, Gly-Lys-Thr, Gly-Lys-Thr-Ala, Gly-Lys-Thr-Cys($R^1$), Asn, Asn-Gly, Asn-Gly-Lys, Lys-Ala, Lys-Ala-Ala, Asn-Gly-Lys-Thr, Asn-Gly-Lys-Thr-Ala, Cys, Cys-Asn, Cys-Asn-Gly, Arg, Arg-Thr, Arg-Thr-Ala, Ser, Cys-Asn-Gly-Lys, Cys-Asn-Gly-Lys-Thr, Cys-Asn-Gly-Lys-Thr-Ala, Ser-Ala, Tyr, Tyr-Cys, Tyr-Cys-Asn, Tyr-Cys-Asn-Gly, Tyr-Cys-Asn-Gly-Lys, Gln, Gln-Ser, Gln-Ser-Ala, Tyr-Cys-Asn-Gly-Lys-Thr, Tyr-Cys-Asn-Gly-Lys-Thr-Ala, Asp, Asp-Tyr, Asp-Tyr-Cys, Asp-Tyr-Cys-Asn, Asp-Tyr-Cys-Asn-Gly, Ile, Ile-Ser, Ile-Ser-Ala, Asp-Tyr-Cys-Asn-Gly-Lys, Asp-Tyr-Cys-Asn-Gly-Lys-Thr, Arg-Ser, Arg-Ser-Ala, Asp-Tyr-Cys-Asn-Gly-Lys-Thr-Ala, Asp-Asp, Asp-Asp-Tyr, Asp-Asp-Tyr-Cys, Asp-Asp-Tyr-Cys-Asn, Asp-Asp-Tyr-Cys-Asn-Gly, Asp-Asp-Tyr-Cys-Asn-Gly-Lys, Asp-Asp-Tyr-Cys-Asn-Gly-Lys-Thr, Asp-Asp-Tyr-Cys-Asn-Gly-Lys-Thr-Ala, Met, Met-Asp, Met-Asp-Asp, Met-Asp-Asp-Tyr, Met-Asp-Asp-Tyr-Cys, Met-Asp-Asp-Tyr-Cys-Asn, Met-Asp-Asp-Tyr-Cys-Asn-Gly, Met-Asp-Asp-Tyr-Cys-Asn-Gly-Lys, Met-Asp-Asp-Tyr-Cys-Asn-Gly-Lys-Thr, Met-Asp-Asp-Tyr-Cys-Asn-Gly-Lys-Thr-Ala, Asp-Met, Asp-Met-Asp, Asp-Met-Asp-Asp, Asp-Met-Asp-Asp-Tyr, Asp-Met-Asp-Asp-Tyr-Cys, Asp-Met-Asp-Asp-Tyr-Cys-Asn, Asp-Met-Asp-Asp-Tyr-Cys-Asn-Gly, Asp-Met-Asp-Asp-Tyr-Cys-Asn-Gly-Lys, Asp-Met-Asp-Asp-Tyr-Cys-Asn-Gly-Lys-Thr, Asp-Met-Asp-Asp-Tyr-Cys-Asn-Gly-Lys-Thr-Ala, A-Cys($R^1$)-Pro, A-Cys($R^1$)-Pro-Arg, A-Cys($R^1$)-Pro-Arg-Asn, A-Cys($R^1$)-Pro-Arg-Asn-Pro, A-Cys($R^1$)-Pro-Arg-Asn-Pro-His, A-Cys($R^1$)-Pro-Arg-Asn-Pro-His-Lys, A-Cys($R^1$)-Pro-Arg-Asn-Pro-His-Lys-Gly, A-Cys($R^1$)-Pro-Arg-Asn-Pro-His-Lys-Gly-Pro, A-Cys($R^1$)-Pro-Arg-Asn-Pro-His-Lys-Gly-Pro-Ala,

wobei A und R$^1$ den in Anspruch 1 angegebenen Definitionen entsprechen, bedeutet
und

X   die angegebene Bedeutung besitzt, aber ungleich Wasserstoff ist, sofern A und damit M fehlen, mit einer Aminoverbindung der Formel III

$$H\text{-}Q\text{-}Z \qquad\qquad III,$$

worin

Z   die angegebene Bedeutung besitzt und

Q   einen Aminosäure- oder Peptidrest, ausgewählt aus einer Gruppe bestehend aus B, Cys(R$^1$)-B, Arg-Asn, Arg-Asn-Pro, Asn-Pro, Arg-Asn-Pro-His, Asn-Pro-His, Pro-His, Arg-Asn-Pro-His-Lys, Asn-Pro-His-Lys, Pro-His-Lys, His-Lys, Arg-Asn-Pro-His-Lys-Gly, Asn-Pro-His-Lys-Gly, Pro-His-Lys-Gly, His-Lys-Gly, Lys-Gly, Arg-Asn-Pro-His-Lys-Gly-Pro, Asn-Pro-His-Lys-Gly-Pro, Pro-His-Lys-Gly-Pro, His-Lys-Gly-Pro, Lys-Gly-Pro, Gly-Pro, Arg-Asn-Pro-His-Lys-Gly-Pro-Ala, Asn-Pro-His-Lys-Gly-Pro-Ala, Pro-His-Lys-Gly-Pro-Ala, His-Lys-Gly-Pro-Ala, Lys-Gly-Pro-Ala, Gly-Pro-Ala, Pro-Ala, Arg-Asn-Pro-His-Lys-Gly-Pro-Ala-Thr, Asn-Pro-His-Lys-Gly-Pro-Ala-Thr, Pro-His-Lys-Gly-Pro-Ala-Thr, His-Lys-Gly-Pro-Ala-Thr, Lys-Gly-Pro-Ala-Thr, Gly-Pro-Ala-Thr, Pro-Ala-Thr, Ala-Thr, Gly-Asp-Cys(R$^1$)-B, Thr-Gly-Asp-Cys(R$^1$)-B, Asp-Cys(R$^1$)-B, Ala-Asp-Cys(R$^1$)-B, Thr-Ala-Asp-Cys(R$^1$)-B, Lys-Thr-Ala-Asp-Cys(R$^1$)-B, Gly-Lys-Thr-Ala-Asp-Cys(R$^1$)-B, Asn-Gly-Lys-Thr-Ala-Asp-Cys(R$^1$)-B, Asn-Cys(R$^1$)-B, Ala-Asn-Cys(R$^1$)-B, Thr-Ala-Asn-Cys(R$^1$)-B, Cys-Asn-Gly-Lys-Thr-Ala-Asp-Cys(R$^1$)-B, Ala-Ala-Asp-Cys(R$^1$)-B, Ser-Ala-Asp-Cys(R$^1$)-B, Tyr-Cys-Asn-Gly-Lys-Thr-Ala-Asp-Cys(R$^1$)-B, Gly-Cys(R$^1$)-B, Ala-Gly-Cys-(R$^1$)-B, Ser-Ala-Gly-Cys(R$^1$)-B, Cys(Trt)-Asp-Cys(R$^1$)-B, Thr-Cys(Trt)-Asp-Cys(R$^1$)-B, Lys-Thr-Cys(Trt)-Asp-Cys(R$^1$)-B, Asp-Tyr-Cys-Asn-Gly-Lys-Thr-Ala-Asp-Cys(R$^1$)-B, Asp-Asp-Tyr-Cys-Asn-Gly-Lys-Thr-Ala-Asp-Cys(R$^1$)-B oder Met-Asp-Asp-Tyr-Cys-Asn-Gly-Lys-Thr-Ala-Asp-Cys(R$^1$)-B

bedeutet,
umsetzt,
und/oder daß man eine freie Mercapto-, Hydroxy- oder Aminogruppe alkyliert
und/oder eine Verbindung der Formel I nach Anspruch 1 durch Behandeln mit einer Säure oder Base in eines ihrer Salze überführt.

5.  Verfahren zur Herstellung pharmazeutischer Zubereitungen, dadurch gekennzeichnet, daß man eine Verbindung der Formel I nach Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

6.  Pharmazeutische Zubereitung, gekennzeichnet durch einen Gehalt an mindestens einer Verbindung der allgemeinen Formel I nach Anspruch 1 und/oder einem ihrer physiologisch unbedenklichen Salze.

7.  Verwendung von Verbindungen der Formel I nach Anspruch 1 oder von deren physiologisch unbedenklichen Salzen zur Herstellung eines Arzneimittels zur Bekämpfung von Krankheiten.

8.  Verwendung von Verbindungen der Formel I nach Anspruch 1 zur Herstellung von immobilisierten Liganden für die Affinitätssäulenchromatographie.

9.  Verwendung von Verbindungen der Formel I nach Anspruch 1 zur Reinigung von Integrinen durch Affinitätschromatographie.

## Claims

1.  Linear peptides of the formula I having a minimum length of 4 amino acid residues

$$X\text{-}A\text{-}Cys(R^1)\text{-}B\text{-}Z \qquad\qquad I,$$

in which

X   is H or Ac,

A   is absent or is Asp or a peptide fragment selected from a group consisting of Ala-Asp, Thr-Ala-Asp, Lys-Thr-Ala-Asp, Lys-Thr-Ala-Asn, Lys-Thr-Gly-Asp, Lys-Ala-Ala-Asp, Arg-Thr-Ala-Asp, Ser-Ala-Asp, Gln-Ser-Ala-Asp, Gly-Lys-Thr-Ala-Asp, Asn-Gly-Lys-Thr-Ala-Asp, Ile-Ser-Ala-Gly, Arg-Ser-Ala-Gly, Cys-Asn-Gly-Lys-Thr-Ala-Asp; Tyr-Cys-Asn-Gly-Lys-Thr-Ala-Asp, Asp-Tyr-Cys-Asn-Gly-Lys-Thr-Ala-Asp, Gly-Lys-Thr-Cys-Asp, Asp-Asp-Tyr-Cys-Asn-Gly-Lys-Thr-Ala-Asp, Gly-Lys-Thr-Cys(Trt)-Asp, Met-Asp-Asp-Tyr-Cys-Asn-Gly-Lys-Thr-Ala-Asp and Asp-Met-Asp-Asp-Tyr-Cys-Asn-Gly-Lys-Thr-Ala-Asp,

B   is absent or is Ala, Arg, Asn, Asp, Cys, Gln, Glu, Gly, His, Ile, Leu, Lys, Met, Orn, Phe, 4-Hal-Phe, Pro, Ser, Thr, Trp, Tyr or Val or is an N-methylated derivative of the amino acid residues mentioned, or is a peptide fragment selected from a group consisting of Pro-Arg, Pro-Arg-Asn, Pro-Arg-Asn-Pro, Pro-Arg-Asn-Pro-His, Pro-Arg-Asn-Pro-His-Lys, Pro-Arg-Asn-Pro-His-Lys-Gly, Pro-Arg-Asn-Pro-His-Lys-Gly-Pro, Pro-Arg-Asn-Pro-His-Lys-Gly-Pro-Ala and Pro-Arg-Asn-Pro-His-Lys-Gly-Pro-Ala-Thr,

in which only one of the residues A or B can be absent,

Z   is OH, $OR^2$, $NH_2$, $NHR^2$ or $NR_2^2$,

$R^1$   is H, $R^2$, Trt, Dpm or Bzl,

$R^2$   is alkyl of 1-6 carbon atoms,

Hal   is F, Cl, Br or I and

Ac   is alkanoyl of 1-10 carbon atoms, aralkanoyl of 8-10 carbon atoms or aroyl of 7-11 carbon atoms,

and their physiologically acceptable salts.

2.   An enantiomer or a diastereomer of a compound of the formula I according to Claim 1.

3.   (a) H-Ala-Asp-Cys(Trt)-Pro-Arg-Asn-Pro-His-Lys-Gly-Pro-Ala-Thr-OH;
     (b) H-Lys-Thr-Ala-Asp-Cys(Trt)-Pro-Arg-Asn-Pro-His-Lys-Gly-Pro-Ala-Thr-OH;
     (c) H-Gly-Lys-Thr-Ala-Asp-Cys(Trt)-Pro-Arg-Asn-Pro-His-Lys-Gly-OH;
     (d) H-Lys-Thr-Ala-Asp-Cys(Trt)-Pro-Arg-Asn-Pro-His-OH;
     (e) H-Lys-Thr-Ala-Asp-Cys(Trt)-Pro-Arg-Asn-Pro-His-Lys-Gly-OH;
     (f) H-Lys-Thr-Ala-Asp-Cys(Trt)-Pro-Arg-OH;
     (g) H-Lys-Thr-Ala-Asp-Cys(Trt)-Pro-OH and
     (h) $H_3$C-CO-Asp-Cys(Trt)-Pro-Arg-Asn-Pro-His-Lys-OH.

4.   Process for the preparation of a compound of the formula I according to Claim 1 or of one of its salts, characterized in that it is liberated from one of its functional derivatives by treatment with a solvolysing or hydrolysing agent or in that a compound of the formula II

$$X\text{-}M\text{-}OH \qquad\qquad II,$$

in which

M  is an amino acid residue or peptide radical selected from a group consisting of A, A-Cys($R^1$), Ala, Thr, Thr-Ala, Lys, Lys-Thr, Lys-Thr-Ala, Gly, Lys-Thr-Ala-Gly, Gly-Lys, Gly-Lys-Thr, Gly-Lys-Thr-Ala, Gly-Lys-Thr-Cys($R^1$), Asn, Asn-Gly, Asn-Gly-Lys, Lys-Ala, Lys-Ala-Ala, Asn-Gly-Lys-Thr, Asn-Gly-Lys-Thr-Ala, Cys, Cys-Asn, Cys-Asn-Gly, Arg, Arg-Thr, Arg-Thr-Ala, Ser, Cys-Asn-Gly-Lys, Cys-Asn-Gly-Lys-Thr, Cys-Asn-Gly-Lys-Thr-Ala, Ser-Ala, Tyr, Tyr-Cys, Tyr-Cys-Asn, Tyr-Cys-Asn-Gly, Tyr-Cys-Asn-Gly-Lys, Gln, Gln-Ser, Gln-Ser-Ala, Tyr-Cys-Asn-Gly-Lys-Thr, Tyr-Cys-Asn-Gly-Lys-Thr-Ala, Asp, Asp-Tyr, Asp-Tyr-Cys, Asp-Tyr-Cys-Asn, Asp-Tyr-Cys-Asn-Gly, Ile, Ile-Ser, Ile-Ser-Ala, Asp-Tyr-Cys-Asn-Gly-Lys, Asp-Tyr-Cys-Asn-Gly-Lys-Thr, Arg-Ser, Arg-Ser-Ala, Asp-Tyr-Cys-Asn-Gly-Lys-Thr-Ala, Asp-Asp, Asp-Asp-Tyr, Asp-Asp-Tyr-Cys, Asp-Asp-Tyr-Cys-Asn, Asp-Asp-Tyr-Cys-Asn-Gly, Asp-Asp-Tyr-Cys-Asn-Gly-Lys, Asp-Asp-Tyr-Cys-Asn-Gly-Lys-Thr, Asp-Asp-Tyr-Cys-Asn-Gly-Lys-Thr-Ala, Met, Met-Asp, Met-Asp-Asp, Met-Asp-Asp-Tyr, Met-Asp-Asp-Tyr-Cys, Met-Asp-Asp-Tyr-Cys-Asn, Met-Asp-Asp-Tyr-Cys-Asn-Gly, Met-Asp-Asp-Tyr-Cys-Asn-Gly-Lys, Met-Asp-Asp-Tyr-Cys-Asn-Gly-Lys-Thr, Met-Asp-Asp-Tyr-Cys-Asn-Gly-Lys-Thr-Ala, Asp-Met, Asp-Met-Asp, Asp-Met-Asp-Asp, Asp-Met-Asp-Asp-Tyr, Asp-Met-Asp-Asp-Tyr-Cys, Asp-Met-Asp-Asp-Tyr-Cys-Asn, Asp-Met-Asp-Asp-Tyr-Cys-Asn-Gly, Asp-Met-Asp-Asp-Tyr-Cys-Asn-Gly-Lys, Asp-Met-Asp-Asp-Tyr-Cys-Asn-Gly-Lys-Thr, Asp-Met-Asp-Asp-Tyr-Cys-Asn-Gly-Lys-Thr-Ala, A-Cys($R^1$)-Pro, A-Cys($R^1$)-Pro-Arg, A-Cys($R^1$)-Pro-Arg-Asn, A-Cys($R^1$)-Pro-Arg-Asn-Pro, A-Cys($R^1$)-Pro-Arg-Asn-Pro-His, A-Cys($R^1$)-Pro-Arg-Asn-Pro-His-Lys, A-Cys($R^1$)-Pro-Arg-Asn-Pro-His-Lys-Gly, A-Cys($R^1$)-Pro-Arg-Asn-Pro-His-Lys-Gly-Pro, A-Cys($R^1$)-Pro-Arg-Asn-Pro-His-Lys-Gly-Pro-Ala,

in which A and $R^1$ are as defined in Claim 1,
and

X is as defined but is not hydrogen if A and therefore M are absent, is reacted with an amino compound of the formula III

$$H-Q-Z \hspace{10em} III,$$

in which

Z is as defined and
Q is an amino acid residue or peptide radical selected from a group consisting of
B, Cys($R^1$)-B, Arg-Asn, Arg-Asn-Pro, Asn-Pro, Arg-Asn-Pro-His, Asn-Pro-His, Pro-His, Arg-Asn-Pro-His-Lys, Asn-Pro-His-Lys, Pro-His-Lys, His-Lys, Arg-Asn-Pro-His-Lys-Gly, Asn-Pro-His-Lys-Gly, Pro-His-Lys-Gly, His-Lys-Gly, Lys-Gly, Arg-Asn-Pro-His-Lys-Gly-Pro, Asn-Pro-His-Lys-Gly-Pro, Pro-His-Lys-Gly-Pro, His-Lys-Gly-Pro, Lys-Gly-Pro, Gly-Pro, Arg-Asn-Pro-His-Lys-Gly-Pro-Ala, Asn-Pro-His-Lys-Gly-Pro-Ala, Pro-His-Lys-Gly-Pro-Ala, His-Lys-Gly-Pro-Ala, Lys-Gly-Pro-Ala, Gly-Pro-Ala, Pro-Ala, Arg-Asn-Pro-His-Lys-Gly-Pro-Ala-Thr, Asn-Pro-His-Lys-Gly-Pro-Ala-Thr, Pro-His-Lys-Gly-Pro-Ala-Thr, His-Lys-Gly-Pro-Ala-Thr, Lys-Gly-Pro-Ala-Thr, Gly-Pro-Ala-Thr, Pro-Ala-Thr, Ala-Thr, Gly-Asp-Cys($R^1$)-B, Thr-Gly-Asp-Cys($R^1$)-B, Asp-Cys($R^1$)-B, Ala-Asp-Cys($R^1$)-B, Thr-Ala-Asp-Cys($R^1$)-B, Lys-Thr-Ala-Asp-Cys($R^1$)-B, Gly-Lys-Thr-Ala-Asp-Cys($R^1$)-B, Asn-Gly-Lys-Thr-Ala-Asp-Cys($R^1$)-B, Asn-Cys($R^1$)-B, Ala-Asn-Cys($R^1$)-B, Thr-Ala-Asn-Cys($R^1$)-B, Cys-Asn-Gly-Lys-Thr-Ala-Asp-Cys($R^1$)-B, Ala-Ala-Asp-Cys($R^1$)-B, Ser-Ala-Asp-Cys($R^1$)-B, Tyr-Cys-Asn-Gly-Lys-Thr-Ala-Asp-Cys($R^1$)-B, Gly-Cys($R^1$)-B, Ala-Gly-Cys($R^1$)-B, Ser-Ala-Gly-Cys($R^1$)-B, Cys(Trt)-Asp-Cys($R^1$)-B, Thr-Cys(Trt)-Asp-Cys($R^1$)-B, Lys-Thr-Cys(Trt)-Asp-Cys($R^1$)-B, Asp-Tyr-Cys-Asn-Gly-Lys-Thr-Ala-Asp-Cys($R^1$)-B, Asp-Asp-Tyr-Cys-Asn-Gly-Lys-Thr-Ala-Asp-Cys($R^1$)-B or Met-Asp-Asp-Tyr-Cys-Asn-Gly-Lys-Thr-Ala-Asp-Cys($R^1$)-B

in which B and $R^1$ are as defined in Claim 1,
and/or in that a free mercapto, hydroxyl or amino group is alkylated
and/or a compound of the formula I according to Claim 1 is converted into one of its salts by treatment with an acid or base.

5. Process for the preparation of pharmaceutical formulations, characterized in that a compound of the formula I according to Claim 1 and/or one of its physiologically acceptable salts, together with at least one solid, liquid or semiliquid excipient or auxiliary, is brought into a suitable administration form.

6. Pharmaceutical formulation, characterized in that it contains at least one compound of the general formula I according to Claim 1 and/or one of its physiologically acceptable salts.

7. Use of compounds of the formula I according to Claim 1 or of physiologically acceptable salts thereof for the preparation of a medicament for combating diseases.

8. Use of compounds of the formula I according to Claim 1 for the preparation of immobilized ligands for affinity column chromatography.

9. Use of compounds of the formula I according to Claim 1 for the purification of integrins by affinity chromatography.

**Revendications**

1. Peptides linéaires de formule I, ayant une longueur minimale de 4 résidus aminoacide

$$\text{X-A-Cys(R}^1\text{)-B-Z} \qquad\qquad \text{I,}$$

formule dans laquelle

X représente H ou Ac,

A est absent ou représente Asp ou un fragment peptidique choisi dans un ensemble constitué par Ala-Asp, Thr-Ala-Asp, Lys-Thr-Ala-Asp, Lys-Thr-Ala-Asn, Lys-Thr-Gly-Asp, Lys-Ala-Ala-Asp, Arg-Thr-Ala-Asp, Ser-Ala-Asp, Gln-Ser-Ala-Asp, Gly-Lys-Thr-Ala-Asp, Asn-Gly-Lys-Thr-Ala-Asp, Ile-Ser-Ala-Gly, Arg-Ser-Ala-Gly, Cys-Asn-Gly-Lys-Thr-Ala-Asp; Tyr-Cys-Asn-Gly-Lys-Thr-Ala-Asp, Asp-Tyr-Cys-Asn-Gly-Lys-Thr-Ala-Asp, Gly-Lys-Thr-Cys-Asp, Asp-Asp-Tyr-Cys-Asn-Gly-Lys-Thr-Ala-Asp, Gly-Lys-Thr-Cys(Trt)-Asp, Met-Asp-Asp-Tyr-Cys-Asn-Gly-Lys-Thr-Ala-Asp, Asp-Met-Asp-Asp-Tyr-Cys-Asn-Gly-Lys-Thr-Ala-Asp,

B est absent ou représente Ala, Arg, Asn, Asp, Cys, Gln, Glu, Gly, His, Ile, Leu, Lys, Met, Orn, Phe, 4-Hal-Phe, Pro, Ser, Thr, Trp, Tyr, Val ou un dérivé N-méthylé des résidus aminoacide cités, ou un fragment peptidique choisi dans un ensemble constitué par Pro-Arg, Pro-Arg-Asn, Pro-Arg-Asn-Pro, Pro-Arg-Asn-Pro-His, Pro-Arg-Asn-Pro-His-Lys, Pro-Arg-Asn-Pro-His-Lys-Gly, Pro-Arg-Asn-Pro-His-Lys-Gly-Pro, Pro-Arg-Asn-Pro-His-Lys-Gly-Pro-Ala, Pro-Arg-Asn-Pro-His-Lys-Gly-Pro-Ala-Thr,

un seul des restes A et B pouvant être absent,

Z représente OH, OR$^2$, NH$_2$, NHR$^2$ ou NR$_2^2$,

R$^1$ représente H, R$^2$, Trt, Dpm ou Bzl,

R$^2$ représente un groupe alkyle ayant de 1 à 6 atomes de carbone,

Hal représente F, Ci, Br ou I, et

Ac représente un groupe alcanoyle ayant de 1 à 10 atomes de carbone, aralcanoyle ayant de 8 à 10 atomes de carbone ou aroyle ayant de 7 à 11 atomes de carbone,

ainsi que leurs sels physiologiquement acceptables.

**2.** Enantiomère ou diastéréoisomère d'un composé de formule I selon la revendication 1.

**3.**     (a) H-Ala-Asp-Cys(Trt)-Pro-Arg-Asn-Pro-His-Lys-Gly-Pro-Ala-Thr-OH,
    (b) H-Lys-Thr-Ala-Asp-Cys(Trt)-Pro-Arg-Asn-Pro-His-Lys-Gly-Pro-Ala-Thr-OH,
    (c) H-Gly-Lys-Thr-Ala-Asp-Cys(Trt)-Pro-Arg-Asn-Pro-His-Lys-Gly-OH,
    (d) H-Lys-Thr-Ala-Asp-Cys(Trt)-Pro-Arg-Asn-Pro-His-OH,
    (e) H-Lys-Thr-Ala-Asp-Cys(Trt)-Pro-Arg-Asn-Pro-His-Lys-Gly-OH,
    (f) H-Lys-Thr-Ala-Asp-Cys(Trt)-Pro-Arg-OH,
    (g) H-Lys-Thr-Ala-Asp-Cys(Trt)-Pro-OH,
    (h) H$_3$C-CO-Asp-Cys(Trt)-Pro-Arg-Asn-Pro-His-Lys-OH.

**4.** Procédé pour la préparation d'un composé de formule I selon la revendication 1 ou d'un de ses sels, caractérisé en ce qu'on le libère à partir d'un de ses dérivés fonctionnels par traitement par un agent de solvolyse ou d'hydrolyse, ou en ce que l'on fait réagir un composé de formule II

$$\text{X-M-OH} \qquad\qquad \text{II,}$$

dans laquelle

M représente un résidu aminoacide ou peptidique choisi dans un ensemble constitué par A, A-Cys(R$^1$), Ala, Thr, Thr-Ala, Lys, Lys-Thr, Lys-Thr-Ala, Gly, Lys-Thr-Ala-Gly, Gly-Lys, Gly-Lys-Thr, Gly-Lys-Thr-Ala, Gly-Lys-Thr-Cys(R$^1$), Asn, Asn-Gly, Asn-Gly-Lys, Lys-Ala, Lys-Ala-Ala, Asn-Gly-Lys-Thr, Asn-Gly-Lys-Thr-Ala, Cys, Cys-Asn, Cys-Asn-Gly, Arg, Arg-Thr, Arg-Thr-Ala, Ser, Cys-Asn-Gly-Lys, Cys-Asn-Gly-Lys-Thr, Cys-Asn-Gly-Lys-Thr-Ala, Ser-Ala, Tyr, Tyr-Cys, Tyr-Cys-Asn, Tyr-Cys-Asn-Gly, Tyr-Cys-Asn-Gly-Lys, Gln, Gln-Ser, Gln-Ser-Ala, Tyr-Cys-Asn-Gly-Lys-Thr, Tyr-Cys-Asn-Gly-Lys-Thr-Ala, Asp, Asp-Tyr, Asp-Tyr-Cys, Asp-Tyr-Cys-Asn, Asp-Tyr-Cys-Asn-Gly, Ile, Ile-Ser, Ile-Ser-Ala, Asp-Tyr-Cys-Asn-Gly-Lys, Asp-Tyr-Cys-Asn-Gly-Lys-Thr, Arg-Ser, Arg-Ser-Ala, Asp-Tyr-Cys-Asn-Gly-Lys-Thr-Ala, Asp-Asp, Asp-Asp-Tyr, Asp-Asp-Tyr-Cys, Asp-Asp-Tyr-Cys-Asn, Asp-Asp-Tyr-Cys-Asn-Gly, Asp-Asp-Tyr-Cys-Asn-Gly-Lys, Asp-Asp-Tyr-Cys-Asn-Gly-Lys-Thr, Asp-Asp-Tyr-Cys-Asn-Gly-Lys-Thr-Ala, Met, Met-Asp, Met-Asp-Asp, Met-Asp-Asp-Tyr, Met-Asp-Asp-Tyr-Cys, Met-Asp-Asp-Tyr-Cys-Asn, Met-Asp-Asp-Tyr-Cys-Asn-Gly, Met-Asp-Asp-Tyr-Cys-Asn-Gly-Lys, Met-Asp-Asp-Tyr-Cys-Asn-Gly-Lys-Thr, Met-Asp-Asp-Tyr-Cys-Asn-Gly-Lys-Thr-Ala, Asp-Met, Asp-Met-Asp, Asp-Met-Asp-Asp, Asp-Met-Asp-Asp-Tyr, Asp-Met-Asp-Asp-Tyr-Cys, Asp-Met-Asp-Asp-Tyr-Cys-Asn, Asp-Met-Asp-Asp-Tyr-Cys-Asn-Gly, Asp-Met-Asp-Asp-Tyr-Cys-Asn-Gly-Lys, Asp-Met-Asp-Asp-Tyr-Cys-Asn-Gly-Lys-Thr, Asp-Met-Asp-Asp-Tyr-Cys-Asn-Gly-Lys-Thr-Ala, A-Cys($R^1$)-Pro, A-Cys($R^1$)-Pro-Arg, A-Cys($R^1$)-Pro-Arg-Asn, A-Cys($R^1$)-Pro-Arg-Asn-Pro, A-Cys($R^1$)-Pro-Arg-Asn-Pro-His, A-Cys($R^1$)-Pro-Arg-Asn-Pro-His-Lys, A-Cys($R^1$)-Pro-Arg-Asn-Pro-His-Lys-Gly, A-Cys($R^1$)-Pro-Arg-Asn-Pro-His-Lys-Gly-Pro, A-Cys($R^1$)-Pro-Arg-Asn-Pro-His-Lys-Gly-Pro-Ala,

A et $R^1$ correspondant aux définitions données dans la revendication 1, et

X a la signification donnée, mais est différent d'un atome d'hydrogène lorsque A et par conséquent M sont absents,

avec un composé aminé de formule III

$$H\text{-}Q\text{-}Z \qquad\qquad III$$

dans laquelle

Z a la signification donnée et
Q représente un résidu aminoacide ou peptidique choisi dans un ensemble constitué par
B, Cys($R^1$)-B, Arg-Asn, Arg-Asn-Pro, Asn-Pro, Arg-Asn-Pro-His, Asn-Pro-His, Pro-His, Arg-Asn-Pro-His-Lys, Asn-Pro-His-Lys, Pro-His-Lys, His-Lys, Arg-Asn-Pro-His-Lys-Gly, Asn-Pro-His-Lys-Gly, Pro-His-Lys-Gly, His-Lys-Gly, Lys-Gly, Arg-Asn-Pro-His-Lys-Gly-Pro, Asn-Pro-His-Lys-Gly-Pro, Pro-His-Lys-Gly-Pro, His-Lys-Gly-Pro, Lys-Gly-Pro, Gly-Pro, Arg-Asn-Pro-His-Lys-Gly-Pro-Ala, Asn-Pro-His-Lys-Gly-Pro-Ala, Pro-His-Lys-Gly-Pro-Ala, His-Lys-Gly-Pro-Ala, Lys-Gly-Pro-Ala, Gly-Pro-Ala, Pro-Ala, Arg-Asn-Pro-His-Lys-Gly-Pro-Ala-Thr, Asn-Pro-His-Lys-Gly-Pro-Ala-Thr, Pro-His-Lys-Gly-Pro-Ala-Thr, His-Lys-Gly-Pro-Ala-Thr, Lys-Gly-Pro-Ala-Thr, Gly-Pro-Ala-Thr, Pro-Ala-Thr, Ala-Thr, Gly-Asp-Cys($R^1$)-B, Thr-Gly-Asp-Cys($R^1$)-B, Asp-Cys($R^1$)-B, Ala-Asp-Cys($R^1$)-B, Thr-Ala-Asp-Cys($R^1$)-B, Lys-Thr-Ala-Asp-Cys($R^1$)-B, Gly-Lys-Thr-Ala-Asp-Cys($R^1$)-B, Asn-Gly-Lys-Thr-Ala-Asp-Cys($R^1$)-B, Asn-Cys($R^1$)-B, Ala-Asn-Cys($R^1$)-B, Thr-Ala-Asn-Cys($R^1$)-B, Cys-Asn-Gly-Lys-Thr-Ala-Asp-Cys($R^1$)-B, Ala-Ala-Asp-Cys($R^1$)-B, Ser-Ala-Asp-Cys($R^1$)-B, Tyr-Cys-Asn-Gly-Lys-Thr-Ala-Asp-Cys($R^1$)-B, Gly-Cys($R^1$)-B, Ala-Gly-Cys($R^1$)-B, Ser-Ala-Gly-Cys($R^1$)-B, Cys(Trt)-Asp-Cys($R^1$)-B, Thr-Cys(Trt)-Asp-Cys($R^1$)-B, Lys-Thr-Cys(Trt)-Asp-Cys($R^1$)-B, Asp-Tyr-Cys-Asn-Gly-Lys-Thr-Ala-Asp-Cys($R^1$)-B, Asp-Asp-Tyr-Cys-Asn-Gly-Lys-Thr-Ala-Asp-Cys($R^1$)-B et Met-Asp-Asp-Tyr-Cys-Asn-Gly-Lys-Thr-Ala-Asp-Cys($R^1$)-B

et/ou en ce que l'on soumet à une alkylation un groupe mercapto, hydroxyle ou amino libre,
et/ou on convertit un composé de formule I selon la revendication 1 en un de ses sels, par traitement par un acide ou une base.

5. Procédé pour la préparation de compositions pharmaceutiques, caractérisé en ce que l'on met sous une forme d'administration appropriée un composé de formule I selon la revendication 1 et/ou un de ses sels physiologiquement acceptables, conjointement avec au moins un adjuvant ou véhicule solide, liquide ou semi-liquide.

6. Composition pharmaceutique, caractérisée par une teneur en au moins un composé de formule générale I selon la revendication 1 et/ou un de ses sels physiologiquement acceptables.

7. Utilisation de composés de formule I selon la revendication 1 ou de leurs sels physiologiquement acceptables, pour la fabrication d'un médicament destiné à la lutte contre des maladies.

8. Utilisation de composés de formule I selon la revendication 1 pour la fabrication de ligands immobilisés destinés à la chromatographie par affinité sur colonne.

9. Utilisation de composés de formule I selon la revendication 1 pour la purification d'intégrines par chromatographie par affinité.